# EUROPÄISCHE PATENTSCHRIFT

(11) **EP 0 736 519 B1**
(45) Veröffentlichungstag und Bekanntmachung des Hinweises auf die Patenterteilung: **03.03.1999**
(21) Anmeldenummer: 96104871.7
(22) Anmeldetag: 27.03.1996
(51) Int. Cl.: C07C 209/86

(54) **Fraktionierung und Reinigung von aromatischen Polyamingemischen und deren Verwendung**
Fractionation and purification of mixtures of aromatic polyamines and their use
Fractionnement et purification de mélanges de polyamines aromatiques et leur utilisation

(30) Priorität: 07.04.1995 DE 19513119
(43) Veröffentlichungstag der Anmeldung: 09.10.1996
(73) Patentinhaber: BAYER AG, 51368 Leverkusen (DE)
(72) Erfinder: Knöfel, Hartmut, Dr., 51519 Odenthal (DE); Brockelt, Michael, 51379 Leverkusen (DE)

(56) Entgegenhaltungen:
- EP-A- 0 031 423
- EP-A- 0 161 600
- EP-A- 0 288 892
- DE-A- 1 568 087
- DE-A- 2 528 694

## Beschreibung

Die Erfindung betrifft ein Verfahren zur Fraktionierung und Reinigung von aromatischen Polyamingemischen und deren Verwendung.

Die Herstellung von aromatischen Polyaminen und Polyamingemischen, insbesondere der Diphenylmethanreihe, wird in zahlreichen Patentmeldungen und Patenten beschrieben, ebenso die Verwendung dieser Produkte. Herausragende Bedeutung kommt dabei der Verwendung dieser Produkte als Rohstoffe für die Herstellung von Isocyanaten zu, in der Regel durch Umsetzung der Polyamingemische mit Phosgen nach den allgemein üblichen und bekannten Methoden.

Die dabei resultierenden Isocyanate bzw. Isocyanatgemische fallen in vielen Fallen aber nicht in der Form und Zusammensetzung an, wie sie auf der Isocyanatstufe bevorzugt weiterverwendet werden, sondern müssen zuvor durch teilweise aufwendige Aufarbeitungs- und Trennverfahren in die verwendungsgerechte Form übergeführt werden. Geeignete Polyaminvorstufen, die weniger aufwendig in die Isocyanatverwendungsformen gebracht werden können, sind in vielen Fällen verfahrenstechnisch schwierig oder gar nicht zugänglich oder wirtschaftlich unattraktiv herzustellen.

Beispielhaft ist die Gewinnung des für die Herstellung hochwertiger Polyurethanwerkstoffe wichtigen 4,4'-Diisocyanato-diphenylmethans, dessen Aminvorstufe in der Regel aus Anilin und Formaldehyd nur gemeinsam mit Isomeren, insbesondere dem 2,4'-Isomeren, und höherfunktionellen Polyaminen gewonnen werden kann. Diese Bestandteile sind zwar die Grundlage für ebenfalls begehrte Isocyanate, doch ist die Auftrennung der Rohisocyanate in die für die Weiterverwendung geeigneten Isocyanate bzw. Isocyanatgemische nicht einfach.

In der Regel werden zunächst ein Teil der Zweikernverbindungen von dem Rest abgetrennt. Anschließend wird aus der Zweikernfraktion in einem viele Trennstufen erfordernden zweiten Destillationsschritt das 4,4'-Diisocyanato-diphenylmethan von den anderen Isomeren befreit.

Das 2,4'-Isomere in angereicherter Form hat selbst in neuerer Zeit zunehmende Bedeutung als Polyurethanrohstoff erlangt, und kann nur mit beträchtlichem destillativen Aufwand gegenüber dem 4,4'-Isomeren angereichert und von dem gegebenenfalls vorhandenen 2,2'-Isomeren befreit werden.

Isomerentrennverfahren oder Anreicherungsverfahren innerhalb der Fraktion der höherkernigen Homologen bzw. der höherfunktionellen Bestandteile der Amine wie auch der Isocyanate der Diphenylmethanreihe sind praktisch nicht bekannt.

Zunehmendes Interesse findet auch das 4,4'-Diamino-diphenylmethan als Rohstoff für das Di-(4-isocyanatocyclohexyl)-methan, die kernhydrierte Form des 4,4'-Diisocyanato-diphenylmethans, wobei die Bereitstellung geeigneter aromatischer Polyamingemische für die Hydrierstufe mit einem möglichst hohen Gehalt an 4,4'-Diamino-diphenylmethan bei gleichzeitig einem möglichst geringen Anteil an 2,4'-Diamino-diphenylmethan sehr aufwendig ist.

Es ist bekannt, daß Amine durch partielle Überführung in ihre Salze in bestimmten Fällen getrennt werden können, wobei u.a. die unterschiedlichen Basenstärken genutzt werden. Dabei handelt es sich in der Regel um Monoamine mit stark unterschiedlichen Basenstärken.

Auch für aromatische Polyamingemische, insbesondere der Diphenylmethanreihe, sind solche Disproportionierungseffekte in zweiphasigen Systemen bereits beschrieben (DE-A 2238319 und DE-A 2528694).

Die Effekte sind infolge der in einem solchen Gemisch vorhanden zahlreichen Komponenten, deren Aminogruppen sich vom Typ her -praktisch alle sind Arylaminogruppen - kaum unterscheiden, nicht besonders groß und ausgeprägt, um für eine direkte Nutzung mit einfachen Mitteln interessant zu sein.

Diese Aufgabe konnte durch das erfindungsgemäße Verfahren, welches aufgrund der erfindungsgemäßen Ausführung überraschend hohe Trennleistung bei der Fraktionierung von aromatischen Polyamingemischen, insbesondere der Diphenylmethanreihe, erzielt, und dabei in der Wirkung weit über die bekannten Effekte des Standes der Technik hinausgeht, gelöst werden.

Bei der erfindungsgemäßen Fraktionierung von aromatischen Polyamingemischen werden andere, unterschiedlich zusammengesetzte Polyamingemische gewonnen.

Bei diesen abgeleiteten Polyamingemischen kann es sich um solche handeln, die auf bekannten Synthesewegen nur sehr aufwendig zugänglich sind. Dabei kann es sich auch um Polyamingemische handeln, die für eine vereinfachte Herstellung der Isocyanate besser geeignet sind, als die bekannten und technisch gut herzustellenden Polyamingemische, indem sie z.B. auf der Isocyanatstufe schwierig durchzuführende Isomerentrennungen auf der Aminstufe vorwegnehmen. Solche Gemische können auch völlig neuartige, weil nach dem Stand der Technik nicht darstellbare Polyamingemische sein, die zu völlig neuartigen Isocyanaten führen.

Andererseits kann das erfindungsgemäße Verfahren dazu genutzt werden, aus beliebigen, d.h. auch aus durch Recycling von Polyurethankunststoffen wiedergewonnenen Polyamingemischen, die sich durch Verunreinigung oder durch nichtstatistische, d.h. selektive Verluste bei einzelnen Komponenten bei der Wiedergewinnung von den ursprünglich eingesetzten Polyaminen oder Isocyanaten unterscheiden, Produktfraktionen zu gewinnen, welche dem Standard oder den Ausgangspolyaminen entsprechen.

Schließlich kann das erfindungsgemäße Verfahren dazu genutzt werden, synthesebedingte und im Endprodukt unerwünschte Neben-und Zwischenprodukte, mitzufraktionieren und in einer Produktfraktion ab - und in einer anderen entsprechend anzureichern, gegebenenfalls in einer eigenen Fraktion auszuschleusen.

Es handelt sich bei der vorliegenden Erfindung um ein breit anwendbares Verfahren, mit welchem die Aufgabe der Fraktionierung und Reinigung von aromatischen Di- und Polyamingemischen, insbesondere der Diphenylmethanreihe, gelöst werden kann.

Aufgabe war es, ein Verfahren zur Verfügung zu stellen, das es gestattet, aromatische Polyamingemische in einfacher Weise zu fraktionieren bzw. zu reinigen, so daß Isomere in reiner Form oder in angereicherter Form anfallen.

Gegenstand der Erfindung ist ein Verfahren zur Fraktionierung und Reinigung von aromatischen Polyamingemischen, insbesondere von Polyamingemischen der Diphenylmethanreihe, welches dadurch gekennzeichnet ist, daß man
a) das Polyaminausgangsgemisch (A) in einem zweiphasigen System, bestehend aus (i) einer hydrophoben Lösungsmittelphase (B), die im wesentlichen aus aromatischem Hilfsamin, welches in Wasser wenig löslich ist und unten Normaldruck einen mindestens 20°C unter dem Siedepunkt der am niedrigsten siedenden Komponente des Ausgangsgemisches liegenden Siedepunkt aufweist, und gegebenenfalls Polyaminen besteht, und (ii) einer wäßrigen Phase (C), bestehend im wesentlichen aus wäßriger Lösung einer starken Säure und gegebenenfalls zumindest teilweise in der Salzform vorliegendem Hilfsamin, und/oder gegebenenfalls zumindest teilweise in der Salzform vorliegenden Polyaminen unter Zuhilfenahme einer nach dem Gegenstromprinzip arbeitenden Extraktionsstufe (7) unter Durchmischung der Phasen verteilt, indem man das Ausgangspolyamingemisch vorzugsweise über die wäßrige Phase (C) in die Extraktionsstufe (7) einbringt, mit der Maßgabe, daß in diesem zweiphasigen System die in den Mengenströmen (A), (B) und (C) eingebrachten Aminäquivalente die Anzahl der im Mengenstrom (C) eingebrachten Säureäquivalente stets übersteigt, und die diese Extraktionsstufe verlassende orgänische Phase (D)
b) gegebenenfalls zumindest teilweise über eine zwischengeschaltete Extraktionsstufe (6) und/oder
c) gegebenenfalls unter Abtrennung eines Teilstromes vor oder nach der gegebenenfalls durchlaufenen Extraktionsstufe (6) und Rückführung des abgetrennten Teilstromes über eine vorgeschaltete Extraktionsstufe (5) zumindest teilweise zur Extraktionsstufe (7)
d) nach Durchlaufen einer Waschstufe und/oder Neutralisationsstufe (10), in einer gegebenenfalls mehrstufig durchgeführten Destillationsstufe (11) in eine Destillatfraktion, bestehend im wesentlichen aus Hilfsamin und eine als Destillationsrückstand (G) anfallende erste Polyaminfraktion auftrennt,
e) die die Extraktionsstufe (7) verlassende wäßrige Phase (H) in eine Neutralisationsstufe (8) leitet, mit Basen, vorzugsweise wäßriger Natronlauge, die in der wäßrigen Phase enthaltene Säure neutralisiert und anschließend in einem Phasentrennschritt in eine wäßrige Phase, enthaltend die Säure in Form ihrer neutralen Salze und eine organische Phase, enthaltend im wesentlichen Polyamin und Hilfsamin, mechanisch auftrennt und
f) die in der Neutralisationsstufe (8) anfallende organische Phase (J) gegebenenfalls nach Durchlaufen einer Waschstufe (9), zumindest teilweise in einer gegebenenfalls mehrstufigen Destillationsstufe (12) aufarbeitet in eine Destillatfraktion (K), enthaltend im wesentlichen Hilfsamin, und in eine als Destillationsrückstand (L) anfallende zweite Polyaminfraktion.

Bevorzugt wird das Verfahren so durchgeführt, daß man
b) die in der Extraktionsstufe (7) anfallende organische Phase (D) zumindest teilweise in einer zwischengeschalteten Extraktionsstufe (6) mit zumindest einer Teilmenge, der wäßrigen Säure (Mengenstrom X) und/oder gegebenenfalls Wasser aus Mengenstrom (Y) und/oder gegebenenfalls Hilfsamin im Gegenstrom extrahiert und/oder mit zumindest einer Teilmenge, vorzugsweise mit der Gesamtmenge der in der gegebenenfalls vorhandenen vorgeschalteten Extraktionsstufe (5) anfallenden wäßrigen Phase (Q) im Gegenstrom extrahiert, die in der zwischengeschalteten Extraktionsstufe (6) resultierende wäßrige Phase (N) der Extraktionsstufe (7) zuführt und die in der zwischengeschalteten Extraktionsstufe (6) anfallende organische Phase (O) der Aufarbeitungsstufe (11) zuführt.

Das erfindungsgemäße Verfahren wird besonders bevorzugt so durchgeführt, daß man
c) einen Teilstrom der die Extraktionsstufe (7) verlassenden organischen Phase (D) und/oder einen Teilstrom der die gegebenenfalls vorhandene zwischengeschaltete Extraktionsstufe (6) verlassenden organischen Phase (O) abtrennt und in einer vorgeschalteten Extraktionsstufe (5) mit zumindest einer Teilmenge der als Mengenstrom (X) zur Verfügung stehenden wäßrigen Säure einstufig umsetzt, vorzugsweise mehrstufig im Gegenstrom extrahiert, den in der Extraktionsstufe (5) eingesetzten organischen Mengenstrom (P) so bemißt daß in (5) ein möglichst weitgehender Übergang des in besagtem organischen Mengenstrom (P) enthaltenen Polyamins in die wäßrige Phase (Q) erfolgt, die in der vorgeschalteten Extraktionsstufe (5) resultierende wäßrige Phase (Q) gegebenenfalls nach Zugabe von Wasser aus Mengenstrom (Y) und/oder Hilfsamin der Extraktionsstufe (6) zuführt und die in der vorgeschalteten Extraktionsstufe (5) anfallende an Polyamin verarmte organische Phase (R) zumindest teilweise der Extraktionsstufe (7) zuführt.

Bei den zum Einsatz gelangenden Hilfsaminen handelt es sich im allgemeinen um Monoanamine wie Anilin und/oder Substituenten tragende Anilinderivate. Bei den Substituenten handelt es sich vorzugsweise um C1-C12- Alkylsubstituenten und/oder Benzylreste am Kern und/oder am Stickstoff des Anilingrundkörpers. Diese Substanzen können sowohl in reiner Form als auch in Form von Isomerengemischen als auch in Form von technischen oder gezielt hergestellten Gemischen untereinander eingesetzt werden.

Geeignete Amine sind beispielsweise: N-Propylanilin, N,N-Dipropylanilin, N-Butylanilin, N,N-dibutylanilin, N-Isobutylanilin, 2-Methylanilin, 2,4-Dimethylanilin, N,2-Dimethylanilin, N,N,2-Trimethylanilin, N-Ethyl-2-methylanilin, 3-Methylanilin, N,N,3-Trimethylanilin, N-Ethyl-3-methylanilin, N,N-Diethyl-3-methylanilin, N-Butyl-3-methylanilin, 3-Trifluormethylanilin, 4-Methylanilin, N,4-Dimethylanilin, N,N,4-Trimethylanilin, N-Ethyl-4-methylanilin, N,N-Diethyl-4-methylanilin, 2-Ethylanilin, 4-Ethylanilin, Xylidine, 2-Isopropylanilin, 2-Ethyl-6-methylanilin, 2,4,5-Trimethylanilin, 2,3,5-Trimethylanilin,4-tert.-Butylanilin, 2-Ethyl-4,6-dimethylanilin, 2,6-Diethyl-4-methylanilin, 2,6-Diisopropylanilin, 4-Cyclohexylanilin, 4-Cyclohexyl-2-methylanilin, 2-Methoxyanilin, 2-Methoxy-N,N-dimethylanilin, 2-Trifluormethylanilin, 2-Ethoxyanilin, 3-Methoxyanilin, Ethoxyanilin, 3-Ethoxy-N,N-diethylanilin, 4-Methoxyanilin, N-Methyl-p-anisidin, 5-Methoxy-2-methylanilin, 2-Methoxy-5-methylanilin, 2-Ethoxy-5-methylanilin, 2,4-Dimethoxyanilin, 2,5-Dimethoxyanilin, 5,6,7,8-Tetrahydro-naphthylamin(1 oder 2).

Bevorzugt als Hilfsamine werden eingesetzt Anilin, 2,6-Dimethylanilin, 2,6-Diethylanilin, 2-Methyl-6-ethylanilin, Mesidin, N-Methylanilin, N-Ethylanilin, N,N-Dimethylanilin, N,N-Diethylanilin, Amino-diphenylmethan.

Als Polyamingemische der Diphenylmethanreihe werden bevorzugt solche eingesetzt, wie sie bei der säurekatalysierten Anilin-/Formaldehyd-Kondensation anfallen.

Die derart behandelten Polyamingemische, also die mit dem erfindungsgemäßen Verfahren erzeugten Fraktionen werden zur Herstellung der entsprechenden aromatischen Polyisocyanatgemische und zur Herstellung von Polyurethankunststoffen verwendet.

Außerdem können die nach dem erfindungsgemäßen Verfahren erzeugten Fraktionen zur Herstellung der entsprechenden kernhydrierten Polyamine oder als Vernetzer und als Epoxidhärter verwendet werden.

Die aus den fraktionierten Polyamingemischen hergestellten entsprechenden Polyisocyanate werden bevorzugt zur Herstellung von PU-Schaumstoffen eingesetzt.

Ausgangsgemische sind beispielsweise technische Arylamingemische, wie sie bei der Herstellung aus den Ausgangsverbindungen oder wie sie bei der Wiedergewinnung anfallen.

Beispiele von Ausgangsarylamingemischen, für deren Fraktionierung und Reinigung das erfindungsgemäße Verfahren besonders geeignet ist, sind
1. Polyamingemische der Diphenylmethanreihe, wie sie bei der Kondensation und säurekatalysierten Umlagerung von Anilin mit Formaldehyd entstehen,
2. Polyamingemische der Diphenylmethanreihe, wie sie bei der säurekatalysierten Kondensation von substituierten Anilinen mit Formaldehyd anfallen,
3. Polyamingemische der Diphenylmethanreihe, wie sie bei der Mischkondensation von substituierten Anilinen untereinander und/oder mit Anilin mit Formaldehyd anfallen,
4. Polyamingemische der Diphenylmethanreihe, wie sie bei der Kondensation, auch der Mischkondensation von substituierten Anilinen und/oder Anilin mit Aldehyden und/oder Ketonen anfallen,
5. Polyamingemische der Diphenylmethanreihe, wie sie bei der Nitrierung und anschließenden Reduktion von Di- und/oder Polyarylmethanen und/oder substituierten Di- und/oder Polyarylmethanen entstehen; unter Polyarylmethanen werden hier insbesondere die Benzylhomologen des Diphenylmethans verstanden,
6. Polyamingemische der Diphenylmethanreihe, wie sie bei der Kondensation von Monoarylmonoaminen (z.B. Anilin, substituierte Aniline) und/oder Monoaryldiaminen (Phenylendiamine, substituierte Phenylendiamine) mit Aldehyden, Ketonen, insbesondere Formaldehyd, und säurekatalysierter Umlagerung entstehen und
7. Polyamingemische der Triphenylmethanreihe, wie sie z.B.bei der Nitrierung und anschließenden Reduktion von Triphenylmethan, insbesondere alkylsubstituierten Triphenylmethanen und seinen höherkernigen, insbesondere Benzylhomologen entstehen.

Bei den eingesetzten Säuren handelt es sich uni wasserlösliche Protonsäuren mit einem unter 2,5, vorzugsweise unter 1,5 liegenden pKA-Wert. Beispiele hierfür sind Salzsäure, Bromwasserstoffsäure, Schwefelsäure, Trifluoressigsäure, Methansulfonsäure oder Phosphorsäure. Bevorzugt eingesetzt werden Salzsäure und Schwefelsäure.

Die genannten Säuren können auch im Gemisch mit sauren oder neutralen Salzen derartiger Säuren, wie z.B. den entsprechenden Ammoniumsalzen oder auch den entsprechenden Alkalisalzen eingesetzt werden.

Im allgemeinen liegen die genannten Säuren in der wäßrigen Phase (C) vor entweder als wäßrige Lösung der freien Säure oder als wäßrige Lösung, die neben der freien Säure auch die Ammoniumsalze der Säure mit Hilfsamin und/oder Polyamin enthält oder als wäßrige Lösung, in der die Säure vollständig in der Form ihrer Ammoniumsalze mit Hilfsamin und/oder Polyamin vorliegt und die gegebenenfalls noch weiteres, nicht salzartig gebundenes Hilfsamin enthält.

Spätestens nach Durchlaufen der Extraktionsstufe (7) liegen die genannten Säuren in der wäßrigen Phase in der Form der Ammoniumsalze der Säure mit der in der wäßrigen Phase befindlichen Fraktion des Polyamins und mit Hilfsamin vor.

Nach Durchlaufen der Extraktionsstufe, gegebenenfalls der Extraktionsstufen, wird die in der wäßrigen Phase befindliche Säure durch Neutralisation mit starken Basen in die entsprechenden Neutralsalze übergeführt. Dabei werden die salzartig gebundenen Polyamine und Hilfsamin freigesetzt.

Das erfindungsgemäße Verfahren kann sowohl diskontinuierlich als auch kontinuierlich durchgeführt werden. Bevorzugte Ausführungsform ist die kontinuierliche Arbeitsweise. Dabei wird das Verfahren in allen Stufen unter dem Eigendruck des Systems und vorzugsweise in einer Inertgasatmosphäre (Stickstoff) durchgeführt.

Das erfindungsgemäße Verfahren kann sowohl mit einer (Abb. 1) als auch mit zwei (Abb. 2 und Abo.3) oder drei (Abb.4) Extraktionsstufen durchgeführt werden.

Das erfindungsgemäße Verfahren kann zur Steigerung des Anreicherungs- bzw. korrespondierenden Abreicherungseffektes mit jeder der anfallenden Produktfraktionen wiederholt werden.

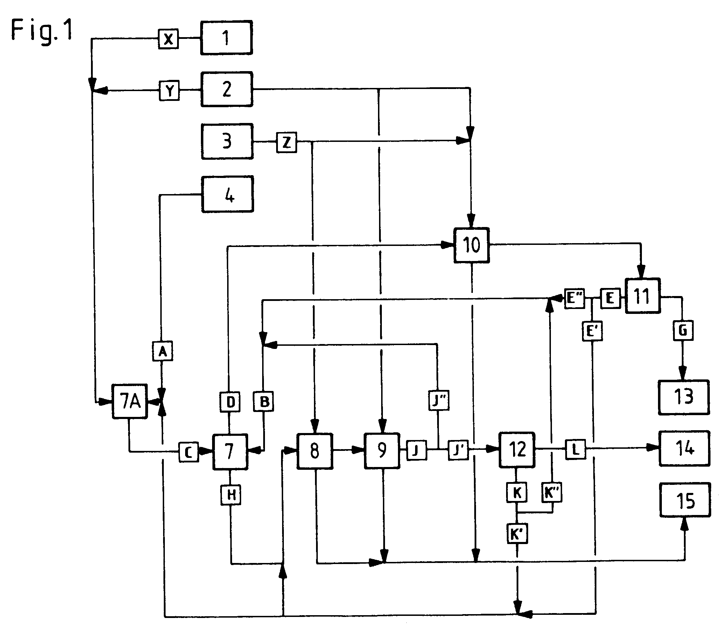

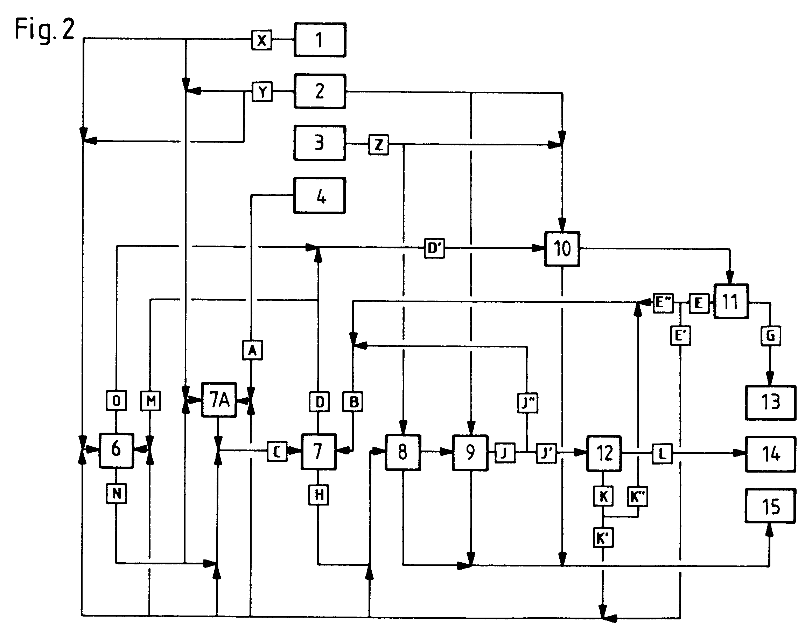

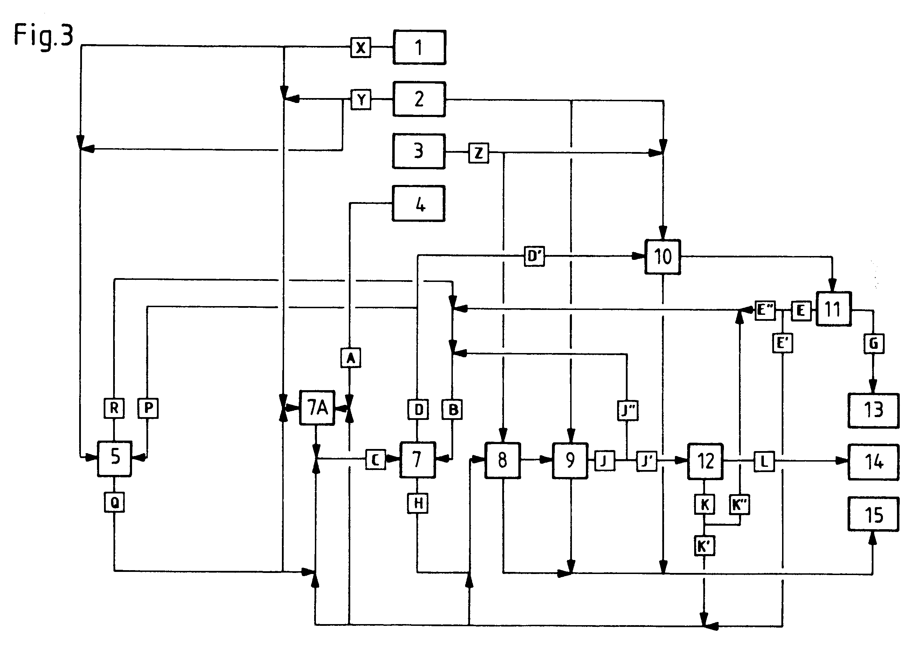

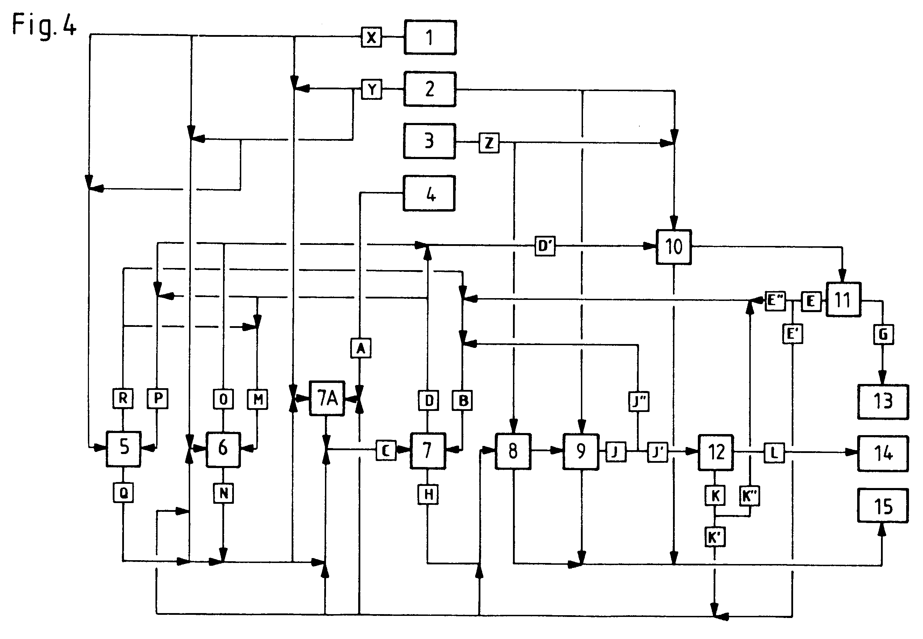

Die in Abb. 1 bis 4 dargestellten Fließdiagramme dienen der weiteren Erläuterung des erfindungsgemäßen Verfahrens. In diesen Abbildungen bedeuten:
(1) einen Tank für wäßrige Säure
(2) einen Tank für Wasser
(3) einen Tank für wäßrige Base
(4) einen Tank für Ausgangspolyamin
(5) in der Regel einen ein- oder mehrstufigen Extraktor, dessen aus der Sicht der wäßrigen Phase erste und gegebenenfalls einzige Stufe aus einer Mischer-Scheidereinheit, im Extremfall nur aus einem Mischer besteht
(6) eine (zwischengeschaltete) Extraktionsstufe
(7A) einen Mischer
(7) eine (weitere) Extraktionsstufe
(8) eine Neutralisationsstufe
(9) eine Waschstufe
(10) eine Wasch- und/oder eine Neutralisationsstufe
(11) eine erste gegebenenfalls mehrstufig betriebene Destillationsstufe
(12) eine weitere gegebenenfalls mehrstufig betriebene Destillationsstufe
(13) einen Tank für ein erstes Verfahrensprodukt
(14) einen Tank für ein weiteres Verfahrensprodukt
(15) einen Tank für Abwasser

Die Bezugszeichen A - R, X, Y und Z bezeichnen die Mengenströme, auf die nachstehend und in den Beispielen Bezug genommen wird.

Bei der Extraktionsstufe (5) handelt es sich im einfachsten Fall um eine einstufig wirkende Mischer-Scheidereinheit, vorzugsweise kommen jedoch mehrstufig wirkende Extraktionseinheiten zum Einsatz, wobei die aus Sicht des Mengenstromes (X) erste Stufe in der Regel aus einer Mischer-Scheidereinheit besteht.

Bei der Extraktionsstufe (6) handelt es sich im einfachsten Fall uni eine einstufig wirkende Mischer-Scheidereinheit, vorzugsweise kommen jedoch mehrstufig wirkende Extraktionseinheiten zum Einsatz.

Bei der Stufe (7A) handelt es sich um einen Mischer.

Die Extraktionsstufe (7) besteht im einfachsten Falle ebenfalls aus einer Mischer-Scheidereinheit, vorzugsweise kommen jedoch auch hier mehrstufig wirkende Extraktionseinheiten zum Einsatz.

Die mehrstufig wirkenden Extraktionseinheiten können aus mehreren in Serie geschalteten Extraktoren bestehen. Vorzugsweise werden die üblichen Gegenstromextraktionsvorrichtungen verwendet.

Bei der Neutralisationsstufe (8) handelt es sich um eine Vorrichtung zur intensiven Durchmischung der wäßrigen Phasen (H) zur Umsetzung der enthaltenen Säure mit der wäßrigen Lösung einer starken Base (Z) im Überschuß aus Behälter (3) mit der Möglichkeit, Neutralisationswärme abzuführen, und anschließender Abtrennung des Polyamins.

Zum Durchmischen werden im einfachsten Falle ein oder mehrere gerührte Kessel eingesetzt, dabei kann der Mischvorgang durch Mischdüsen, Intensivmischer und/oder Umpumpvorrichtungen verstärkt werden. Für die anschließende Phasentrennung werden im einfachsten Falle Scheider eingesetzt, wobei die Phasentrennung durch den Einbau von Scheidehilfen verstärkt werden kann. Ebenso geeignet sind beispielsweise Zentrifugen.

In den Fällen, in denen nach der Umsetzung der wäßrigen Phasen (H) mit starken Basen die einfache mechanische Abtrennung schwierig oder nicht möglich ist, wird die Abtrennung durch den Einsatz von zusätzlichem Hilfsamin oder gegebenenfalls von Wasser durchgeführt, gegebenenfalls als Extraktionsvorgang in einen vorzugsweise mehrstufig wirkenden Extraktor.

Bei der Waschstufe (9), handelt es sich im einfachsten Fall uni eine Mischer-Scheidereinheit, in welcher der Mengenstrom (J) mit Wasser gewaschen wird, für die Durchführung des erfindungsgemäßen Verfahrens ist die Waschstufe (9) grundsätzlich nicht erforderlich, in der Regel aber vorteilhaft.

Auch bei der Wasch- und/oder Neutralisationsstufe (10) handelt es sich im einfachsten Fall um eine Mischer-Scheidereinheit, vorzugsweise kommen jedoch mehrstufig wirkende Extraktoren zum Einsatz.

In der Verfahrensstufe (10) kann die organische Phase (D) sowohl mit Wasser als auch vorzugsweise mit verdünnten wäßrigen Lösungen starker Basen umgesetzt und Säurefrei gemacht werden.

Bei Durchführung der Verfahrensstufe (10) als reine Waschstufe unter Verwendung von Wasser wird die resultierende wäßrige Phase nach deren Abtrennung nicht dem Abwasser zugeschlagen, sondern an geeigneter Stelle in den Verfahrensablauf zurückgeführt.

Die Destillationsstufen (11) und (12) bestehen im einfachsten Fall aus je einer Destillationskolonne, in welcher das jeweilige Zulaufprodukt in je eine Destillatfraktion, bestehend aus Hilfsamin, und den Destillationsrückstand, bestehend im Falle der Destillationsstufe (11) aus einer ersten Polyaminfraktion (G) und im Falle der Destillationsstufe (12) aus einer zweiten Polyaminfraktion (L).

Der Einsatz einer energetisch günstigeren Mehrstufendestillation in Stufe (11) und/oder (12) ist besonders dann bevorzugt, wenn der damit verbundene technische Mehraufwand wirtschaftlich gerechtfertigt ist. Die anfallenden Destillate können dabei vor ihrer Wiederverwendung vereinigt und miteinander vermischt werden.

Bei Durchführung der Neutralisationsstufe (8) unter Mitverwendung von zusätzlichem Hilfsamin als Lösungsmittel enthält das Zulaufprodukt zur Destillationsstufe (12) in der Regel beträchtliche Anteile an Hilfsamin, die im allgemeinen gemeinsam als Destillatfraktion (K) von der als Destillationsrückstand anfallenden zweiten Polyaminfraktion (L) abgetrennt werden.

Das erfindungsgemäße Verfahren kann in mehreren technischen Varianten durchgeführt werden.

Gemäß einer ersten Variante erfolgt die Einspeisung des Ausgangspolyamingemisches (Mengenstrom A) aus Behälter (4) in die Verfahrensstufe (7).

Die gegebenenfalls vorhandene Stufe (7A) dient der Entlastung der Extraktionsstufe (7) und besteht im einfachsten Fall der ersten Variante des erfindungsgemäßen Verfahrens aus einem der Stufe (7) vorgeschalteten Mischer, in dem aus wäßriger Säure (Mengenstrom X), gegebenenfalls Wasser (Mengenstrom Y) zur Einstellung einer gewünschten Säurekonzentration und/oder gegebenenfalls Hilfsamin der eigentliche Mengenstrom (C) gebildet und eingestellt wird.

Es hat sich als vorteilhaft erwiesen, auch das Ausgangspolyamin (A) zumindest teilweise bereits im Mischer (7A) mit den Bestandteilen des Mengenstromes (C) zu vermischen und die resultierende wäßrige Phase als Mengenstrom (C) der Extraktionsstufe (7) zuzuführen, bestehend in der Regel aus Wasser, einer starken Säure, Polyamin und gegebenenfalls Hilfsamin.

Im allgemeinen liegt die Säure in der wäßrigen Phase (C) vor als wäßrige Lösung der Säure, die gegebenenfalls Ammoniumsalze der Säure mit Polyamin und/oder Hilfsamin enthält; vorzugsweise liegt die Säure vor als wäßrige Lösung ihrer Ammoniumsalze mit Polyamin und/oder Hilfsamin, die gegebenenfalls freies, d.h. nicht salzartig gebundenes Polyamin und/oder Hilfsamin gelöst enthält.

Danach ist es durchaus möglich und zur Lösung eines speziellen Trennproblemes gegebenenfalls auch vorteilhaft, den Mengenstrom (C) der Stufe (7) gänzlich ohne Hilfsamin zuzuführen, sofern die in jeden Fall für die Verfahrensstufe (6) geltende Randbedingung erfüllt ist, wonach die Summe der in den Mengenströmen (A), (B) und (C) eingebrachten Aminäquivalente stets die Anzahl der in Mengenstrom (C) eingebrachten Säureäquivalente übersteigt.

Es hat sich als zweckmäßig erwiesen, den Säuregehalt der wäßrigen Phase unabhängig von dem sich je nach Verfahrensparametern (z.B. Zusammensetzung von organischer und wäßriger Phase, Phasenverhältnis, Temperatur) in der wäßrigen Phase eines zweiphasigen Systems einstellenden Amingehalt über eine sogenannte "Molarität" zu definieren.

Die "Molarität" wird festgelegt als theoretische Konzentration von zu 100% protoniertem Amin (d.h.gleiche Anzahl von Säure und Aminäquivalenten) in einem rechnerisch um den Anteil an nicht protonierten Amin vermindertem Volumen oder gegebenenfalls in einem rechnerisch um eine entsprechende Aminmenge bis zur vollständigen Bindung der Säure als Ammoniumsalze erweitertem Volumen an wäßriger Phase.

Der für die jeweilige Ausführungsform des erfindungsgemäßen Verfahrens wohldefinierte und in engen Grenzen gemessene und geregelte Säuregehalt des Mengenstroms (C) wird als wichtige Führungsgröße je nach der der jeweiligen Ausführungsform zugrunde liegenden - hier produktbezogenen - Trennaufgabe in einem weiten Bereich insgesamt oder in einzelnen Verfahrensstufen gezielt variiert, gegebenenfalls unter Zufuhr von Wasser aus Mengenstrom (Y) oder wäßriger Säure aus Mengenstrom (X).

Nach oben wird dieser Arbeitsbereich praktisch begrenzt einerseits durch zunehmende Kristallisationsneigung der Aminsalze mit zunehmender Konzentration, und andererseits durch die zunehmende gegenseitige Löslichkeit der Phasen ineinander.

Der Arbeitsbereich des erfindungsgemäßen Verfahrens bezüglich Molarität wird nach unten wirtschaftlich begrenzt. Durch den abnehmenden Säuregehalt sinkt die Trennleistung quantitativ, d.h. bei hervorragender qualitativer Trennleistung und technisch problemlos, ist mit sinkender Molarität ein zunehmend größeres Volumen an wäßriger Phase erforderlich für die Trennung einer gegebenen Aminmenge.

In der vorzugsweise mehrstufig betriebenen Extraktionsstufe (7) werden die organische Phase (B) und die wäßrige Phase (C) unter inniger Durchmischung einander entgegengeführt.

Bei diesem Vorgang findet in der Regel ein Übergang von Polyarylamin von der wäßrigen Phase (C) in die organische Phase (B) statt, gegebenenfalls in, Austausch gegen Arylamin in entgegengesetzter Richtung.

In der die Extraktionsstufe (7) verlassenden wäßrigen Phase (H) liegt die Säure als wäßrige Lösung ihrer Ammoniumsalze mit Polyamin und gegebenenfalls Hilfsamin vor, die in der Regel darüber hinaus noch freies, d.h. nicht salzartig gebundenes Polyamin und gegebenenfalls freies, d.h. nicht salzartig gebundenes Hilfsamin gelöst enthält.

Die organische Phase (B) besteht im allgemeinen aus Hilfsamin und/oder Polyamin, letzteres vorzugsweise mit der Zusammensetzung des zweiten Verfahrensteilproduktes (L).

Bei der Durchführung des erfindungsgemäßen Verfahrens wird der Mengenstrom (B) im einfachsten Fall gebildet aus dem Destillatstrom (E), der in diesem Fall gegebenenfalls einstufigen Destillationsstufe (11). Der Mengenstrom (E) besteht in diesem Fall praktisch aus Hilfsamin. Die Wirksamkeit der Verfahrensstufe (7) ist in diesem Falle lediglich an einen genügend großen Überschuß der Aminäquivalente insgesamt über die Säureäquivalente in der eingebrachten wäßrigen Phase geknüpft.

Wegen der erhöhten Kristallisationsneigung der Ammoniumsalze der Polyamine wird die Extraktionsstufe (7) in der Regel bei erhöhten Temperaturen betrieben, vorzugsweise bei Temperaturen oberhalb von 80°C, gegebenenfalls unter Druck.

Das zusammen mit der wäßrigen Phase (C) in den Extraktor (7) eingebrachte Ausgangspolyamin (A) verteilt sich auf die den Extraktor verlassende wäßrige Phase (H) und die den Extraktor (7) verlassende organische Phase (D) (quantitative Fraktionierung).

Die mengenmäßige Aufteilung der einzelnen Komponenten des Ausgangspolyamingemisches auf die resultierende wäßrige Phase (H) und die resultierende organische Phase (D) erfolgt dabei unter den Bedingungen des erfindungsgemäßen Verfahrens mit einer überraschend hohen Selektivität, so daß die resultierenden Produktfraktionen eine andere, unter Umständen stark von der des Ausgangspolyamingemisches abweichende Zusammensetzung aufweisen (qualitative Fraktionierung).

Beispielsweise ausgehend von den bevorzugt eingesetzten Anilinformaldehydkondensationsprodukten wurde gefunden, daß von einer in zwei oder mehreren isomeren Formen im Ausgangsgemisch enthaltenen Polyamikomponente in der Regel die ortho-Isomere(n) Form(en) in der die Trennstufe (7) verlassenden organischen Phase (D) relativ angereichert ist (sind); beispielsweise 2,4'-Diamino-diphenylmethan relativ zu 4,4'-Diamino-diphenylmethan. Umgekehrt ist die resultierende wäßrige Phase (H) relativ verarmt an dem 2,4'-Isomeren, während das 4,4'-Isomere relativ angereichert ist.

Sind mehrere "ortho-Isomere" im Ausgangspolyamin vorhanden, z.B. 2,2'- und 2,4'-Diamino-diphenylmethan, dann ist das "ortho-reichere" 2,2'-Isomere in der organischen Phase (D) gegenüber dem "ortho-ärmeren" 2,4'-Isomeren stärker angereichert, welchletzteres seinerseits gegenüber dem noch "ortho-ärmeren" 4,4'-Isomeren relativ angereichert ist.

Der zuerst bei den Anilinformaldehydkondensationsprodukten der Diamino-diphylmethanreihe gefundene An- und Abreicherungseffekt wurde rein empirisch deskriptiv mit dem Kriterium der ortho- und para-Subtitution verbunden. Die davon abgeleitete Charakterisierung der Verfahrensprodukte als "orthoreich" und "orthoarm" ist dabei relativ und wurde durch den Begriff "ortho-Substitutionsgrad" ausgedrückt.

Als "ortho-Subtitutionsgrad" wird dabei das Verhältnis der orthoständigen Aminogruppen-Methylengruppenrelationen zur Gesamtzahl aller Aminogruppenrelationen definiert. Mit diesem Begriff lassen sich praktisch alle Isomerentrennungen bei den Polyaminen erfassen, die aus Arylaminen, auch substituierten, mit Carbonylverbindungen in wäßrig saurem Medium hergestellt werden.

Überraschenderweise wurde nun der gleiche An- und Abreicherungseffekt - geordnet nach ortho-Substitutionsgrad - auch für die gut charakterisierten und analytisch erfaßbaren isomeren Dreikernverbindungen aus der Anilin-/Formaldehydkondensation gefunden.

Analoges gilt für die Trennung von Kondensationsprodukten aus Formaldehyd mit Anilin und Diaminoarylverbindungen wie Phenylendiamin oder alkylsubstituierten Phenylendiaminen.

Die bisher erwähnten Polyamingemische weisen, bedingt durch ihre Herstellung Aminogruppen auf, die praktisch nur orthoständig und/oder paraständig zu Methylengruppen sind. Dabei werden innerhalb einer Gruppe isomerer Verbindungen in der Regel diejenigen mit den höheren ortho-Substitutionsgrad gegenüber den Isomeren mit einem geringeren ortho-Substitutionsgrad bei der Fraktionierung in der organischen Phase (D) angereichert.

Polyamingemische insbesondere der Diphenylmethanreihe einschließlich der jeweiligen höherkernigen Homologen, die nach anderen Verfahren hergestellt werden, beispielsweise durch Nitrierung von Diphenylmethan oder Methyldiphenylmethanen und anschließende Reduktion weisen außer ortho- und para-ständigen Aminogzuppen herstellungsbedingt auch andere Aminogruppen-Methylengruppenrelationen auf. Für diese Polyamingemische ist das erfindungsgemäße Verfahren genauso wirksam.

Beispielsweise läßt sich aus einem Gemisch von 2- und 4-Methyldiphenylmethan durch Nitrierung und anschließende Reduktion ein Polyamingemisch herstellen, welches in der Hauptsache ein Isomerengemisch darstellt aus

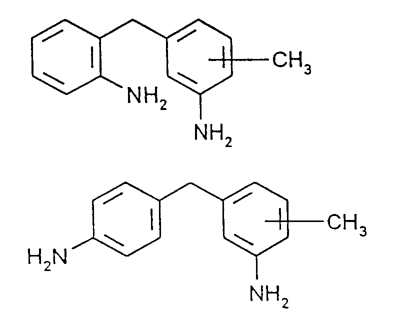

Bei der Fraktionierung solcher Gemische mit Hilfe des erfindungsgemäßen Verfahrens werden die 3,2'-Amino-Isomeren in der organischen Phase (D) gegenüber den 3,4'-Amino-Isomeren angereichert.

Das Kriterium "orthoreich" und "orthoarm" oder der "ortho-Substitutionsgrad" erfaßt in diesen Polyamingemischen nicht mehr alle Isomeren und ist daher sinngemäß anzuwenden, indem anstelle der Begriffe "orthoständig" und "paraständig" eine Einteilung der Isomeren vorgenommen wird in solche mit kleinerem (ortho-) und solche mit größerem (para-) räumlichen Abstand der - in der Regel an unterschiedlichen Sechsringen befindlichen - Aminogruppen zur Methylenbrücke bzw. der Aminogruppen zueinander.

Eine weitere Klasse aromatischer Polyamingemische, die sich mit Hilfe des erfindungsgemäßen Verfahrens sehr wirksam fraktionieren lassen, stellen die Polyamine des Triphenylmethans und seiner höherkernigen Homologen, vorzugsweise Benylhomologen dar, wie sie z.B. durch Nitrierung und anschließende Reduktion der entsprechenden Kohlenwasserstoffgemische hergestellt werden.

Bei der Fraktionierung technischer Polyamingemische der zuletzt genannten Substanzklassen
I. Mischkondensationsprodukte von Mono und Diaminoarylverbindungen mit Formaldehyd bzw. allgemeinen Carbonylverbindungen,
II. Polyamingemische aus Verfahren durch Nitrierung und anschließende Reduktion von Diphenylmethan und vorzugsweise substituierten insbesondere alkylsubstituierte Diphenylmethanen und den jeweiligen Homologen und
III. Polyamingemische aus Verfahren durch Nitrierung und anschließende Reduktion von Triphenylmethan und vorzugsweise substituierten insbesondere alkylsubstituierten Triphenylmethanen und den jeweiligen höherkernigen Benzylhomologen
wurde zusätzlich zur reinen Isomerentrennung eine weitere überaschende Selektivität gefunden.

Polyamingemische der genannten Substanzklasse I bis III enthalten oder können enthalten Komponenten, bei denen wenigstens ein Arylkern pro Molekül mehr als eine, in der Regel zwei Aminogruppen trägt. Diese Komponenten können die bevorzugten Bestandteile des Polyamingemisches sein, ohne daß sie verfahrensbedingt mengenmäßig die Hauptprodukte sein müssen.

Zur besseren Charakterisierung solcher Komponenten wird der Begriff "Aminosubstitutionsgrad" verwendet, mit dem in erster Linie die Anzahl der Aminogruppen einer Komponente im Verhältnis zur Anzahl der Arylkerne gekennzeichnet wird.

Für Anilin und seine Kondensationsprodukte mit Formaldehyd ist dieser Ausdruck stets 1,0 ,für Phenylendiamin und seine Kondensationsprodukte stets 2,0. Für reine Mischkondensate ergeben sich für die Diphenylmethanisomeren der Wert 1,5 und für die höherkernigen Homologen Werte zwischen > 1,0 und < 2,0. Bei statistischer Verwendung des Begriffes Aminosubstitutionsgrad zur Charakterisierung von technischen Polyamingemischen ergeben sich ebenfalls Werte zwischen 1,0 und 2,0.

Bei der Fraktionierung von Polyamingemischen mit einem Aminosubstitutionsgrad > 1,0 wurde nun gefunden, daß die Komponenten mit einem höheren Aminosubstitutionsgrad in der resultierenden wäßrigen Phase (H) relativ angereichert werden, und zwar um so stärker je größer der Aminosubstitutionsgrad ist.

Bei Polyamingemischen, die durch Nitrierung und nachfolgende Reduktion von Polyarylsystemen mit drei oder mehr Arylkernen hergestellt werden, können Polyaminkomponenten mit einen Aminosubstitutionsgrad < 1 gebildet werden, die ebenfalls der Fraktionierung durch das erfindungsgemäße Verfahren unterliegen.

Unabhängig davon ist auch hier die Trennung nach dem "ortho-Substitutionsgrad" wirksam.

Somit eröffnet das erfindungsgemäße Verfahren auch für diese Substanzklasse neue Wege, die Herstellungsform der Rohstoffe (Aminstufe) und die Verwendungsform der Endprodukte (Isocyanatstufe) durch Fraktionierung und/oder Anreicherung auf der Aminstufe und separate Weiterverarbeitung der Fraktionen zu entkoppeln, so daß eine getrennte Optimierung beider Stufen erleichtert wird bis hin zur Gewinnung völlig neuer Isocyanatgemische oder erst möglich wird, wo bislang geeignete Verfahren und Methoden fehlten oder wenig präktikabel sind.

Ergänzt werden diese "Leistungen" durch ein weiteres Selektivitätskriterium, welches bei der Fraktionierung technischer Polyamingemische, insbesondere solcher mit höherkernigen Homologen, gefunden wurde und die "Kernigkeit" der Polyamingemische betrifft.

Mit dem Begriff "Kernigkeit" wird primär die Anzahl der Aryleinheiten einer Komponente eines aromatischen Polyamingemisches ausgedrückt. Im weiteren Sinne wird der Begriff der Kernigkeit dafür verwendet, um für ein aus zahlreichen Komponenten, mit einer individuell exakten aber unterschiedlichen Kernigkeit bestehenden Polyamingemisch statistisch eine Kernigkeit des Gesamtgemisches auszudrücken.

Besonders überraschenderweise wurde nun bei der Fraktionierung von Polyamingemischen mit höherkernigen Anteilen, insbesondere bei der Fraktionierung technischer Gemische von Anilin-Formaldehydkondensaten, gefunden, daß sich solche Gemische auch nach dem Kriterium der Kernigkeit fraktionieren lassen.

Insbesondere führt eine niedrige Molarität der wäßrigen Phase (C) innerhalb des verfahrensmäßig nutzbaren Molaritätsbereichs zu einer relativen Anreicherung höherkerniger Komponenten in der organischen Phase (D).

Der überraschende Befund kann dahingehend erweitert und präzisiert werden daß die relative An- und Abreicherung auch innerhalb der höherkernigen Homologen untereinander stattfindet. Werden beispielsweise in einem technischen Gemisch des Diamino-diphenylmethans in der einen Fraktion die Dreikernkomponenten gegenüber den Zweikernhomponenten relativ an- oder abgereichert, wird auch eine gleichsinnige relative An- oder Abreicherung von Vierkernkomponenten gegenüber Dreikernkomponenten, d.h. eine noch stärkere relative An- oder Abreicherung, gefunden, desgleichen von Fünfkernkomponenten gegenüber Vierkernkomponenten u.s.w.

Daraus und aus der gleichzeitig und stets im Sinne einer relativen Verstärkung des "ortho-Substitutionsgrades" in der organischen Phase (D) ablaufenden Isomerentrennung und aus der Möglichkeit, mit einzelnen Produktfraktionen die erfindungsgemäße Trennung, gegebenenfalls mit geänderten Verfahrensparametern, zu wiederholen, ergeben sich zahlreiche Möglichkeiten, ausgehend von bekannten und gut zugänglichen Polyamingemischen über das erfindungsgemäße Verfahren zu weniger gut zugänglichen oder völlig neuen, weil nach dem Stand der Technik bislang unzugänglichen, Polyaminen und damit Polyisocyanaten zu gelangen. Das gilt besonders für Produkte, der Diamino- und Diisocyanato-diphenylmethanreihe und ganz besonders für Polyamin - und Polyisocyanatgemische mit einem extrem hohen Anteil an höherkernigen Komponenten.

Die An- bzw. Abreicherung wird in der Regel effektiver mit steigendem Protonierungsgrad in der wäßrigen Phase der Trennstufe.

Der Protonierungsgrad gibt das Verhältnis von Säureäquivalenten zu Aminäquivalenten wieder.

Darüber hinaus erweist sich das erfindungsgemäße Verfahren als von allgemeiner Wirksamkeit auch auf andere strukturähnliche Polyamine.

So können beispielsweise in den bereits erwähnten Polyamingemischen, die durch Nitrierung von Di- und Polyarylmethanen und anschließender Reduktion gewonnen werden, auch Monoaminopolyarylmethanverbindungen oder Komponenten enthalten sein, bei denen eine oder mehrere Methylengruppen durch Nebenreaktionen in Keto- und/oder Hydroxymethylengruppen und damit in unerwünschte Nebenprodukte umgewandelt worden sind.

Bei der Kondensation von Arylaminen mit Carbonylverbindungen können zahlreiche unvollständig umgelagerte Zwischenverbindungen und Nebenprodukte auftreten.

Die meisten dieser Verbindungen unterliegen in der Regel bei der Fraktionierung der sie enthaltenden Polyamingemische einer Anreicherung in einer der resultierenden Fraktionen, so daß der Effekt zur Abtrennung und Fraktionierung genutzt werden kann.

Gegebenenfalls können derartige Produkte auf diesem Wege angereichert werden oder als gezielt hergestellte Polyamingemische, wie z.B. Polyaminobenzophenone oder Aminobenzylarylamingemische ihrerseits fraktioniert werden.

Die die Extraktionsstufe (7) verlassende, organische Phase (D) enthält unter anderem in Abhängigkeit vom eingesetzten Hilfsamin wechselnde Mengen an Säure, die vor der destillativen Aufarbeitung des Mengenstromes (D) entfernt werden.

Im einfachsten Falle geschieht dies in der Verfahrensstufe (10) durch Waschen mit Wasser und/oder durch Neutralisation mit überschüssigen, verdünnten wäßrigen Basen, beispielsweise verdünnter Natronlauge.

Die organische Phase (D) bzw. (D') wird nach Durchlaufen der Stufe (10) in die Destillationsstufe (11) überführt.

In der letzten Stufe der gegebenenfalls mehrstufigen Destillationsstufe (11) wird das erste Polyaminteilprodukt (G) abgetrennt und im Verfahrensprodukttank (13) gesammelt.

Das entsprechende zweite Teilprodukt befindet sich in der die Extraktionsstufe (7) verlassenden wäßrigen Phase (H).

Die wäßrige Phase (H) wird gegebenenfalls nach Zusatz von Hilfsamin, in der Neutralisationsstufe (8) mit der wäßrigen Lösung einer starken Base, vorzugsweise Natronlauge, zur Neutralisation der enthaltenen Säure umgesetzt.

Die bei der Neutralisation gebildete wäßrige Phase wird abgetrennt und im Abwasserbehälter (15) gesammelt.

Die bei der Neutralisation gebildete organische Phase wird als Mengenstrom (J) abgetrennt, gegebenenfalls in der Waschstufe (9) mit Wasser gewaschen, und der destillativen Aufarbeitung (12) zugeführt.

In der letzten Stufe der gegebenenfalls mehrstufigen Destillationsstufe (12) wird das zweite Polyaminteilprodukt (L) abgetrennt und im Verfahrensprodukttank (14) gesammelt.

Mit dieser ersten Variante des erfindungsgemäßen Verfahrens lassen sich beträchtlich Trennleistungen bei der Fraktionierung von Polyamingemischen erzielen und zahlreiche Trennprobleme zufriedenstellend lösen.

Insbesondere in der zweiten Polyaminfraktion (L) kann die relative Anreicherung der in dieser Fraktion bevorzugt enthaltenen Komponente gezielt variiert und maximiert werden.

Der in der ersten Polyaminfraktion (G) verbleibende Anteil dieser Komponenten kann jedoch gemäß dieser ersten Variante nicht in gleicher Weise minimiert werden, sondern variabel nur bis zu einem Gehalt relativ abgereichert werden, dessen untere Grenze abhängt von dem für die jeweiligen Verfahrensparameter charakteristischen Verteilungsgleichgewicht der Polyaminkomponenten von (A) zwischen der organischen Phase (D) beim Verlassen des Extraktors (7) und der wäßrigen Phase (C) beim Eintritt in den Extraktor (7).

Die organische Phase (B) besteht im allgemeinen aus Hilfsamin und/oder Polyamin, letzteres vorzugsweise mit der Zusammensetzung des zweiten Verfahrensteilproduktes (L).

Bei der Verwendung einer organischen Phase (B) ohne Polyamin resultiert in der die Extraktionsstufe (7) verlassenden wäßrigen Phase (H) eine Polyaminfraktion, in welcher die relative Anreicherung der in dieser Phase bevorzugt enthaltenen Komponenten gezielt erhöht und maximiert werden kann, auf Kosten der Polyaminkonzentration in der wäßrigen Phase.

Polyamin als Bestandteil der organischen Phase (B) bewirkt, daß die die Verfahrensstufe (7) verlassende Phase (H) eine höhere und damit für die Durchführung des erfindungsgemäßen Verfahrens energetisch vorteilhaftere Polyaminkonzentration aufweist, als bei Verwendung einer organischen Phase (B) ohne Polyamin.

Durch die bevorzugte Verwendung eines Polyamins mit der Zusammensetzung des zweiten Teilproduktes (L) als Bestandteil der organischen Phase (B) kann auch auf einem höheren und damit vorteilhaften Konzentrationsniveau infolge Gleichgewichtseinstellung mit Selbstverstärkung des Trenneffektes die relative Anreicherung der in der die Trennstufe (7) verlassenden wäßrigen Phase (H) bevorzugt enthaltenen Polyaminkomponeten und damit der zweiten Polyaminfraktion (L) variiert und maximiert werden.

Vorteilhafter und als Ausführungsform bevorzugt ist eine zweite Variante des erfindungsgemäßen Verfahrens, bei der sich zusätzlich auch in der ersten Polyaminfraktion (G) die relevante Anreicherung der in dieser Fraktion bevorzugt enthaltenen Komponenten beträchtlich steigern und gezielt variieren läßt, indem die in der Extraktionsstufe (7) anfallende organischen Phase (D) zumindest teilweise in einer aus der Sicht der Phase (D) zwischengeschalteten Extraktionsstufe (6) mit einer wäßrigen Phase extrahiert wird, die im vorliegendem Fall der Variante 2 im wesentlichen aus zumindest einer Teilmenge des Mengenstroms (X) und gegebenenfalls zusätzlichem Wasser aus Mengenstrom (Y) und gegebenenfalls Hilfsamin besteht.

Aus formalen Gründen wird die der Extraktionsstufe (6) zugeführte organische Phase als Mengenstrom (M) bezeichnet, auch wenn sie gegebenenfalls wie im vorliegenden Fall beispielhaft ausgeführt, zumindest in der Zusammensetzung, vorzugsweise aber auch in der Menge mit Mengenstrom (D) übereinstimmt.

Bereits bei der einstufigen Durchführung der Extraktionsstufe (6), beispielsweise als Mischer-Scheidereinheit, kommt es in der resultierenden organischen Phase (O) in Abhängigkeit von Art und Menge der eingesetzten wäßrigen Phase zu einer deutlichen weiteren relativen Anreicherung der bereits in (D) gegenüber Ausgangspolyamin (A) angereicherten Komponenten, verbunden mit einer Abnahme des Polyamingehaltes in der resultierenden organischen Phase (O). Vorzugsweise wird jedoch wegen der besseren Effektivität auch die zwischengeschaltete Extraktionsstufe (6) als mehrstufig wirkender und im Gegenstrom betriebener Extraktor ausgeführt.

Die in der Extraktionsstufe (6) anfallende wäßrige Phase (N) enthält die entsprechende andere Fraktion des mit Mengenstrom (M) eingebrachten Polyamins, in der die in (O) angereicherten Komponenten entsprechend abgereichert sind. Ausmaß der relativen Abreicherung, d.h. die Zusammensetzung des in (O) enthaltenen Polyamins, wird unter den jeweiligen Verfahrensbedingungen der mehrstufig wirkenden Extraktionsstufe (6) kontrolliert durch das qualitative und quantitative Verteilungsgleichgewicht zwischen der zugeführen organischen Phase (M) und der abgeführten wäßrigen Phase (N).

Die Molarität der wäßrigen Phase in der Extraktionsstufe (6) liegt je nach Trennaufgabe höher oder auf gleicher Höhe oder niedriger, bezogen auf die Molarität in der aus Sicht der wäßrigen Phase nachgeschalteten Extraktionsstufe (7) und wird durch die Zugabe von Säure und/oder die Zugabe oder gegebenenfalls den Entzug von Wasser an geeigneter Stelle geregelt.

Die in der Verfahrensstufe (6) resultierende wäßrige Phase (N) wird gegebenenfalls nach Zugabe von Wasser zusammen mit dem gegebenenfalls vorhandenen Rest von (X) der Extraktionsstufe (7) zugeführt.

Die in Stufe (6) resultierende organische Phase (O) wird zusammen mit dem gegebenenfalls vorhandenen Rest von (D) der Destillationsstufe (11) zugeführt zur Gewinnung der Polyaminfraktion (G).

Mit der zweiten Variante des erfindungsgemäßen Verfahrens läßt sich die relative Anreicherung in beiden resultierenden Polyaminfraktionen gezielt variieren und maximieren. Neben dieser in qualitativer Hinsicht großen Vielseitigkeit und Leistungsfähigkeit stellt die zweite Verfahrensvariante zumindest für die zweite Polyaminfraktion (L) auch eine energetisch günstige Ausführungsform dar.

Eine in energetischer Hinsicht für das Teilprodukt (G) verbesserte Ausführung stellt die dritte Variante des erfindungsgemäßen Verfahrens dar. Ausgehend von der ersten Variante wird diese dahingehend erweitert, daß die die Verfahrensstufe (7) verlassende, das erste Teilprodukt (G) in gegenüber der Konzentration von (A) in (C) verringerter Konzentration enthaltende organische Phase (D) geteilt wird in einen Mengenstrom (D'), der weiterhin mit dem Ziel der Gewinnung der Polyaminfraktion (G) den Aufarbeitungsstufen (10) und (11) zugeführt wird, und in einen Mengenstrom (P).

Der Mengenstrom (P) wird in einer vorgelagerten Extraktionsstufe (5) mit zumindest einer Teilmenge, vorzugsweise mit der Gesamtmenge der als Mengenstrom (X) zur Verfügung stehenden wäßrigen Säure umgesetzt; gegebenenfalls erfolgt die Umsetzung als mehrstufige Gegenstromextraktion.

Bei der Extraktionsstufe (5) handelt es sich in der Regel um einen mehrstufig wirkenden und im Gegenstrom betriebenen Extraktor, in welchem die zugeführte organische Phase (P) mit zumindest einer Teilmenge, vorzugsweise mit der Gesamtmenge der zur Wiederverwendung zur Verfügung stehenden wäßrigen Säure (X) extrahiert wird.

Der dem Extraktor (5) zugeführte Mengenstrom (P) wird dabei so bemessen, daß bei der Umsetzung mit Mengenstrom (X) ein möglichst weitgehender, vorzugsweise praktisch quantitativer Übergang zumindest der in der organischen Phase (P) enthaltenen Polyamine in die den Extraktor (5) verlassende wäßrige Phase (Q) erfolgt.

Übersteigt die Summe der in die Verfahrensstufe (5) eingebrachten Säureäquivalente die der Aminäquivalente, erfolgt der Übergang der Amine in die wäßrige Phase bereits in einer einzigen Verfahrensstufe praktisch quantitativ, so daß keine organische Phase (R) resultiert. Das Vorhandensein von freier Säure in der resultierenden wäßrigen Phase ist für den Fortgang des Verfahrens ohne Belang.

Auch im Falle eines Überschusses der Aminäquivalente in (P) über die Säureäquivalente in (X), und sogar bei einem begrenzten Überschuss der Polyaminäquivalente in (P) über die Säureäquivalente in (X), kann eine im Sinne des erfindungsgemäßen Verfahrens an Polyamin ausreichend verarmte organische Phase (R) gewonnen werden durch Mehrstufigkeit der vorgelagerten Verfahrensstufe (5) und Arbeiten im Gegenstrom.

Der Restgehalt an Polyamin in der die Verfahrensstufe (5) resultierenden organische Phase (R) liegt im allgemeinen bei < 3 Gew.%, vorzugsweise bei < 1 Gew.%.

Im übrigen richtet der sich in (R) zulässige Höchstgehalt an Amin und insbesondere der Gehalt an Polyamin nach den aus der jeweiligen Trennaufgabe resultierenden qualitativen Anforderungen an die Verfahrensprodukte, im Falle der Variante 3 insbesondere an das Verfahrensteilprodukt (L). Die Einhaltung des für die Qualität von (L) relevanten Gehaltes an Polyamin wird Im Rahmen der technischen Gegebenheiten unter Ausschöpfung der zur Verfügung stehenden wäßrigen Säure (X), gegebenenfalls einer Menge von (Y), über die Bemessung des Teilmengenstromes (P) kontrolliert.

Dabei kommt es dem Verfahren und insbesondere der Extraktionsstufe (5) zustatten, daß die für den Einsatz in Stufe (5) zur Verfügung stehende wäßrige Säure (Mengenstrom X) um so größer ist, je größer der Anteil der zweiten Polyaminfraktion (L) und je kleiner demzufolge der Anteil der ersten Polyaminfraktion (G) ist. Eine kleine Polyaminfraktion (G) bedeutet in der Regel eine niedrige Polyaminkonzentration in der organischen Phase (D) und hohen Energieaufwand bei der Aufarbeitung einer solchen Phase. Durch die erfindungsgemäße Variante 3 kann gegenüber Variante 1 insbesondere der Energieaufwand bei der Isolierung der ersten Polyaminfraktion (G) reduziert werden.

Der Beitrag der Verfahrensstufe (5) im Rahmen der Variante 3 zur Verbesserung des erfindungsgemäßen Verfahrens besteht darin, daß für die destillative Aufarbeitung (11) zur Gewinnung der ersten Polyaminfraktion (G) anstelle des Gesamtstromes mit einer relativ niedrigen und damit energetisch ungünstigen Konzentration an Polyamin nur ein Teilstrom mit entsprechend erhöhter und damit energetisch günstigerer Konzentration anfällt (quantitative Anreicherung), während aus dem anderen Teilstrom eine an geeigneter Stelle als Extraktionsmittel verwendbare organische Phase (R) ohne Destillation gewonnen wird.

Die von Polyamin weitgehend befreite, die Verfahrensstufe (5) verlassende organische Phase (R) wird der Extraktionsstufe (7) zugeführt.

In der vorzugsweise mehrstufig wirkenden Extraktionsstufe (7) wird die organische Phase (R) als Extraktionsmittel zugesetzt, in der Regel durch Vermischen mit Mengenstrom (B) und Zugabe zu der aus der Sicht der organischen Phase (B) ersten Stufe des Extraktors.

In Abhängigkeit von einem gegebenenfalls vorhandenen Restgehalt an Polyamin in (R) und mit Rücksicht auf die Qualität der zweiten Polyaminfraktion (L) erfolgt die Zugabe der organischen Phase (R) gegebenenfalls zu einer aus Sicht der organischen Phase (B) späteren, gegebenenfalls zur letzten Stufe der mehrstufig arbeitenden Extraktionsstufe (7).

Die die Verfahrensstufe (5) verlassende wäßrige Phase (Q) enthält neben der zumindest teilweise in Form ihrer Ammoniumsalze vorliegenden Säure Polyamin mit einer Zusammensetzung, die weitgehend dem Polyamin in der zugeführten organischen Phase (P) entspricht und gegebenenfalls Hilfsamin.

Im Falle der Variante 3 des erfindungsgemäßen Verfahrens wird der Mengenstrom (Q) direkt der Verfahrensstufe (7) zugeführt, gegebenenfalls nach Zugabe von Wasser aus Mengenstrom (Y) und/oder weiterer wäßriger Säure aus Mengenstrom (X).

Da die in der wäßrigen Phase (Q) enthaltene Polyaminfraktion in der Regel eine höhere relative (qualitative) Anreicherung im Sinne der ersten Polyaminfraktion (G) bezogen auf das Ausgangspolyamin (A) aufweist, resultiert für die der Extraktionsstufe (7) zugeführte wäßrige Phase nach Zugabe von Ausgangspolyamin (A) ein gegenüber diesem in Abhängigkeit vom Mengenverhältnis "angereichertes" Mischpolyamin. Infolge des Verteilungsgleichgewichtes zwischen zugeführter wäßriger und resultierender organischer Phase (D) ergibt daraus für Variante 3 auch ein begrenzter zusätzlicher qualitativer Anreicherungseffekt für die erste Polyaminfraktion (G).

In einer weiteren Variante 4 des erfindungsgemäßen Verfahrens werden die technischen Maßnahmen der vorausgegangenen Varianten zusammengefaßt und miteinander kombiniert.

Im einfachsten Fall werden die Extraktionsstufen (5) und (6) hinzugefügt und jede für sich mit einem Teilstrom von (X), gegebenenfalls einen Teilstrom von (Y) und einem Teilstrom von (D), der in diesem Fall gegebenenfalls in drei Teilströme aufgeteilt wird, in der beschriebenen Weise durchgeführt.

Vorteilhafter ist es, als organische Phase (P) in Extraktionsstufe (5) einen Teilstrom des, eine qualitativ hochangereicherte und quantitativ weniger konzentrierte Polyaminfraktion enthaltenden, Mengenstromes (O) einzusetzen.

Bevorzugt wird die Variante 4 so durchgeführt, daß als organische Phase (P) in der Extraktionsstufe (5) ein Teilstrom von (D) und/oder vorzugsweise ein Teilstrom von (O) eingesetzt wird und die in Stufe (5) resultierende wäßrige Phase (Q) zumindest teilweise, vorzugsweise insgesamt der Extraktionsstufe (6) zugeführt und gegebenenfalls unter Zugabe von weiterer wäßriger Säure aus Mengenstrom (X) und gegebenenfalls von Hilfsamin in (6) eingesetzt wird. Dabei wird ihr unter inniger Durchmischung in mehreren Stufen die organische Phase (M) mit ihrem Gehalt an im gleichen Sinne angereicherten Polyamin entgegengeführt, gegebenenfalls wird die organische Phase (M) vermehrt durch Zugabe eines Teilstromes der in der Extraktionsstufe (5) resultierenden organischen Phase (R) zu (M).

Durch diese Maßnahmen kommt es in der in (6) resultierenden organischen Phase (O) zu einer weiteren Steigerung des qualitativen Anreicherungseffektes. In quantitativer Hinsicht kann dieses Ergebnis durch Bemessung und Aufteilung der Mengenströme bei einem relativ hohen und damit energetisch günstigem Polyamingehalt in den in (6) resultierenden Phasen, insbesondere in der organischen Phase (O) erreicht werden.

Die Rückkopplung des Anreicherungseffektes in (O) über den Mengenstrom (P) als Teilstrom von (O) und über die wäßrige Phase (Q) wirkt dabei selbstverstärkend.

Durch die erfindungsgemäße Ausführung und Verschaltung der Extraktionsstufen (5) bis (7) in Variante (4) mit Verfahrenskriterien wie Disproportionierung anstelle von fraktionierter Extraktion in Stufe (6), mit Selbstverstärkung durch Verschältung mit Extraktionsstufe (5) und Wiedergewinnung von Extraktionsmittel in Stufe (5) ohne Destillation für den Einsatz in der Verfahrensstufe (7) und gegebenenfalls in (6) resultiert ein Maximum an qualitativer Trennleistung, die in Kombination mit der Variation der Molarität der wäßrigen Phasen in den Stufen (5) bis (7) zu einer großen Anwendungsbreite des erfindungsgemäßen Verfahrens führt.

### Beispiele

### Beispiel 1

In einem Mischer (7A) werden das Ausgangspolyamingemisch (Mengenstrom A) (1,900 kg/h) mit 1,400 kg/h 30 %ige Salzsäure (Mengenstrom X) und 2,700 kg/h Wasser (Mengenstrom Y) miteinander vermischt unter Bildung von Mengenstrom (C).

| | |
|---|---|
| Mengenstrom (C) ( 6,000 kg/h ) | 31,7 % Polyarylamin |
| | 7,0 % Chlorwasserstoff |
| | 61,3 % Wasser |

Mengenstrom (C) wird in einem mehrstufig wirkenden Extraktor (7) bei 85°C dem organischen Mengenstrom (B) entgegengeführt,der im wesentlichen aus 2,6-Dimethylanilin besteht.

| |
|---|
| Mengenstrom (B) ( 7,200 kg/h ) |

Die dabei resultierende, die Extraktionsstufe (7) verlassende organische Phase (Mengenstrom D) hat folgende durchschnittliche Zusammensetzung:

| | |
|---|---|
| Mengenstrom (D) ( 7,600 kg/h ) | 12,0 % Polyarylamin |
| | 87,3 % 2,6-Dimethylailin |
| | 0,2 % Chlorwasserstoff |
| | 0,5 % Wasser. |

Mengenstrom (D) wird in der Neutralisationsstufe (10) mit überschüssiger verdünnter Natronlauge (Teilmengenstrom von Z ) und Wasser aus Behälter (2) umgesetzt. Die wäßrige Phase wird als Abwasser in Tank (15) gesammelt.

Der gewaschene und von Säureresten befreite Mengenstrom (D) wird in der nachfolgenden Destillationsstufe (11) aufgetrennt in eine Destillatfraktion (E) mit 6,635 kg/h, die im wesentlichen aus 2,6-Dimethylanilin besteht und einem Destillationsrückstand, der als Mengenstrom (G) mit 0,910 kg/h im Tank (13) gesammelt wird und der die erste Polyaminfraktion darstellt.

Das Destillat (E) wird zur Bildung des Mengenstromes (B) verwendet und der Extraktionsstufe (7) zugeführt.

Die den Extraktor (7) verlassende wäßrige Phase (H) hat folgende Zusammensetzung:

| | |
|---|---|
| Mengenstrom (H) ( 5,600 kg/h ) | 17,7 % Polyarylamin |
| | 10,1 % 2,6-Dimethylailin |
| | 7,2 % Chlorwasserstoff |
| | 65,0 % Wasser. |

Mengenstrom (H) wird in der nachfolgenden Neutralisationsstufe (8) mit überschüssiger wäßriger Natronlauge aus Tank (3) (Hauptmenge von Mengenstrom Z) neutralisiert. Die wäßrige, salzhaltige Phase wird abgetrennt und im Abwassertank (15) gesammelt.

Die organische Phase wird anschließend in der Waschstufe (9) mit Wasser aus Tank (2) salzfrei gewaschen. Das Waschwasser wird ebenfalls im Abwassertank (15) gesammelt.

Die die Waschstufe verlassende organische Phase (Mengenstrom J) wird in der Destillationsstufe (12) aufgetrennt in eine Destillatfraktion (K) und einen Destillationsrückstand (L).

Mengenstrom (K) (0,565 kg/h) besteht im wesentlichen aus 2,6-Dimethylailin und wird der Extraktionsstufe (7) zugeführt zur Bildung von Mengenstrom (B).

Mengenstrom (L) stellt mit 0,990 kg/h die zweite Polyaminfraktion dar und wird im Tank (14) gesammelt.

| **Polyarylamin GC:** | **A** [Gew. - %] | **G** [Gew. - %] | **L** [Gew. %] |
|---|---|---|---|
| 2,2' Diamino-diphenylmethan | 0,3 | 0,6 | ---- |
| 2,4'-Diamino-diphenylmethan | 5,4 | 11,4 | 0,1 |
| 4,4'-Diamino-diphenylmethan | 62,8 | 52,7 | 69,9 |
| N-Methyl-4,4'-diamino-diphenylmethan | 0,1 | 0,2 | ---- |
| Σ Diamino-diphenylmethane | 68,6 | 64,9 | 72,0 |
| Σ Mehrkernpolyamine | 31,4 | 35,1 | 28,0 |
| Mengenverteilung | 100 % | 48,0 % | 52,0 % |

### Beispiel 2

In einem Mischer (7A) werden das Ausgangspolyamingemisch (Mengenstrom A) (1,445 kg/h) und die den Extraktor (5) verlassende wäßrige Phase (Mengenstrom Q) (6,000 kg/h) miteinander vermischt unter Bildung von Mengenstrom (C).

| | |
|---|---|
| Mengenstrom (C) ( 6,000 kg/h ) | 25,6 % Polyarylamin |
| | 19,3 % 2,6-Dimethylanilin |
| | 5,6 % Chlorwasserstoff |
| | 49,5 % Wasser. |

Mengenstrom (C) wird in einem mehrstufig wirkenden Extraktor (7) bei 85°C dem organischen Mengenstrom (B) entgegengeführt,der im wesentlichen aus 2,6-Dimethylanilin besteht.

Mengenstrom (B) setzt sich zusammen aus Mengenstrom (R), der den Extraktor (5) verlassenden organischen Phase (2,100 kg/h), und den beiden Destillatmengenströmen (E) und (K).

| |
|---|
| Mengenstrom (E)+(K) ( 4,055 kg/h ) |

Dabei wird die aus den Destillaten (E) und (K) bestehende Teilmenge in der aus Sicht der organischen Phase (B) ersten Stufe des insgesamt 10-stufigen Extraktor (7) eingesetzt, und die aus Mengenstrom (R) bestehende Teilmenge wird in der aus Sicht der organischen Phase (B) dritten Stufe des Extraktors (7) zugesetzt.

Die in Stufe (7) resultierende organische Phase (Mengenstrom D) hat folgende durchschnittliche Zusammensetzung:

| | |
|---|---|
| Mengenstrom (D) ( 8,000 kg/h ) | 11,4 % Polyarylamin |
| | 87,9 % 2,6-Dimethylailin |
| | 0,2 % Chlorwasserstoff |
| | 0,5 % Wasser. |

Von der organische Phase (D) wird ein Teilstrom (Mengenstrom P) mit ca. 4,0 kg/h abgetrennt und der vorgeschalteten Extraktionsstufe (5) zugeführt.

Der verbleibende Mengenstrom (D')wird in der Neutralisationsstufe (10) mit überschüssiger verdünnter Natronlauge (Teilmengenstrom von Z) und Wasser aus Behälter (2) umgesetzt. Die resultierende wäßrige Phase wird abgetrennt und als Abwasser in Tank (15) gesammelt.

Der gewaschene und von Säureresten befreite Mengenstrom (D') wird in der nachfolgenden Destillationsstufe (11) aufgetrennt in eine Destillatfraktion (E) mit 3,535 kg/h, die im wesentlichen aus 2,6-Dimethylanilin besteht und einem Destillationsrückstand, der als Mengenstrom (G) mit 0,455 kg/h im Tank (13) gesammelt wird und der die erste Polyaminfraktion darstellt.

Das Destillat (E) wird zur Bildung von Mengenstrom (B) verwendet.

Der von (D) als Mengenstrom (P) abgetrennte Teilstrom wird in einer vorgeschalteten Extraktionsstufe (5) einer wäßrigen Phase entgegengeführt, die aus 1,400 kg/h 30%iger Salzsäure (Mengenstrom X) und 2,700 kg/h Wasser (Mengenstrom Y) gebildet wird.

Die aus der Sicht der wäßrigen Phase erste Stufe, der als mehrstufiger Extraktor ausgeführten Stufe (5), besteht aus einem Mischer aus an Salzsäure beständigem Material mit der Möglichkeit zur Abführung der (Neutralisations-)Wärme. Ansonsten wird die Stufe (5) bei 85.- 90°C durchgeführt.

Die resultierende organische Phase (R) (2,100 kg/h) besteht im wesentlichen aus 2,6-Dimethylanilin und wird zur Bildung von Mengenstrom (B) verwendet.

Die resultierende wäßrige Phase (Q) hat folgende durchschnittliche Zusammensetzung:

| | |
|---|---|
| Mengenstrom (Q) ( 6,000 kg/h ) | 7,6 % Polyarylamin |
| | 23,9 % 2,6-Dimethylailin |
| | 7,1 % Chlorwasserstoff |
| | 61,4 % Wasser |

und wird unter Bildung von Mengenstrom (C) der Stufe (7) zugeführt.

Die den Extraktor (7) verlassende wäßrige Phase (H) hat folgende Zusammensetzung:

| | |
|---|---|
| Mengenstrom (H) ( 5,600 kg/h ) | 17,7 % Polyarylamin |
| | 9,3 % 2,6-Dimethylailin |
| | 7,3 % Chlorwasserstoff |
| | 65,7 % Wasser. |

Mengenstrom (H) wird in der nachfolgenden Neutralisationsstufe (8) mit überschüssiger wäßriger Natronlauge aus Tank (3) (Hauptmenge von Mengenstrom Z) neutralisiert. Die wäßrige, salzhaltige Phase wird abgetrennt und im Abwassertank (15) gesammelt.

Die organische Phase wird anschließend in der Waschstufe (9) mit Wasser aus Tank (2) salzfrei gewaschen. Das Waschwasser wird ebenfalls im Abwassertank (15) gesammelt.

Die die Waschstufe verlassende organische Phase (Mengenstrom J) wird in der Destillationsstufe (12) aufgetrennt in eine Destillatfraktion (K) und einen Destillationsrückstand (L).

Mengenstrom (K) (0,520 kg/h) besteht im wesentlichen aus 2,6-Dimethylailin und wird der Extraktionsstufe (7) zugeführt zur Bildung von Mengenstrom (B).

Mengenstrom (L) stellt mit 0,990 kg/h die zweite Polyaminfraktion dar und wird im Tank (14) gesammelt.

| **Polyarylamin GC:** | **A** [Gew. - %] | **G** [Gew. - %] | **L** [Gew. %] |
|---|---|---|---|
| 2,2' Diamino-diphenylmethan | 0,3 | 1,0 | ---- |
| 2,4'-Diamino-diphenylmethan | 5,4 | 16,7 | 0,2 |
| 4,4'-Diamino-diphenylmethan | 62,8 | 43,2 | 71,8 |
| N-Methyl-4,4'-diamino-diphenylmethan | 0,1 | 0,3 | ---- |
| Σ Diamino-diphenylmethane | 68,6 | 61,2 | 72,0 |
| Σ Mehrkernpolyamine | 31,4 | 38,8 | 28,0 |
| Mengenverteilung | 100 % | 31,5 % | 68,5 % |

## Patentansprüche

1. Verfahren zur Fraktionierung und Reinigung von aromatischen Polyamingemischen der Diphenylmethanreihe dadurch gekennzeichnet, daß man
a) das Polyaminausgangsgemisch (A) in einem zweiphasigen System, bestehend aus (i) einer hydrophoben Lösungsmittelphase (B), die im wesentlichen aus aromatischem Hilfsamin, welches in Wasser wenig löslich ist und unter Normaldruck einen mindestens 20°C unter dem Siedepunkt der am niedrigsten siedenden Komponente des Ausgangsgemisches liegenden Siedepunkt aufweist, besteht, und (ii) einer wäßrigen Phase (C), bestehend im wesentlichen aus wäßriger Lösung einer starken Säure und gegebenenfalls zumindest teilweise in der Salzform vorliegendem Hilfsamin, unter Zuhilfenahme einer nach dem Gegenstromprinzip arbeitenden Extraktionsstufe (7) unter Durchmischung der Phasen verteilt, indem man das Ausgangspolyamingemisch überdie wäßrige Phase (C) in die Extraktionsstufe (7) einbringt, mit der Maßgabe, daß in diesem zweiphasigen System die in den Mengenströmen (A), (B) und (C) eingebrachten Aminäquivalente die Anzahl der im Mengenstrom (C) eingebrachten Säureäquivalente stets übersteigt, und die diese Extraktionsstufe verlassende organische Phase (D)
b) zumindest teilweise über eine zwischengeschaltete Extraktionsstufe (6) und/oder
c) unter Abtrennung eines Teilstromes vor oder nach der durchlaufenen Extraktionsstufe (6) und Rückführung des abgetrennten Teilstromes über eine vorgeschaltete Extraktionsstufe (5) zumindest teilweise zur Extraktionsstufe (7)
d) nach Durchlaufen einer Waschstufe und/oder Neutralisationsstufe (10), in einer Destillationsstufe (11) in eine Destillatfraktion, bestehend im wesentlichen aus Hilfsamin und eine als Destillationsrückstand (G) anfallende erste Polyaminfraktion auftrennt,
e) die die Extraktionsstufe (7) verlassende wäßrige Phase (H) in eine Neutralisationsstufe (8) leitet, mit Basen, die in der wäßrigen Phase enthaltene Säure neutralisiert und anschließend in einem Phasentrennschritt in eine wäßrige Phase, enthaltend die Säure in Form ihrer neutralen Salze und eine organische Phase, enthaltend im wesentlichen Polyamin und Hilfsamin, mechanisch auftrennt und
f) die in der Neutralisationsstufe (8) anfallende organische Phase (J) gegebenenfalls nach Durchlaufen einer Waschstufe (9), zumindest teilweise in einer Destillationsstufe (12) aufarbeitet in eine Destillatfraktion (K), enthaltend im wesentlichen Hilfsamin, und in eine als Destillationsrückstand (L) anfallende zweite Polyaminfraktion.

2. Verfahren gemäß Anspruch 1, dadurch gekennzeichnet, daß man
b) die in der Extraktionsstufe (7) anfallende organische Phase (D) zumindest teilweise in einer zwischengeschalteten Extraktionsstufe (6) mit zumindest einer Teilmenge, der wäßrigen Säure (Mengenstrom X) im Gegenstrom extrahiert und/oder mit der Gesamtmenge der in der gegebenenfalls vorhandenen vorgeschalteten Extraktionsstufe (5) anfallenden wäßrigen Phase (Q) im Gegenstrom extrahiert, die in der zwischengeschalteten Extraktionsstufe (6) resultierende wäßrige Phase (N) der Extraktionsstufe (7) zuführt und die in der zwischengeschalteten Extraktionsstufe (6) anfallende organische Phase (O) der Aufarbeitungsstufe (11) zuführt.

3. Verfahren gemäß Anspruch 1 oder 2, dadurch gekennzeichnet, daß man
c) einen Teilstrom der die Extraktionsstufe (7) verlassenden organischen Phase (D) und/oder einen Teilstrom der die zwischengeschaltete Extraktionsstufe (6) verlassenden organischen Phase (O) abtrennt und in einer vorgeschalteten Extraktionsstufe (5) mit zumindest einer Teilmenge der als Mengenstrom (X) zur Verfügung stehenden wäßrigen Säure einstufig umsetzt, mehrstufig im Gegenstrom extrahiert, den in der Extraktionsstufe (5) eingesetzten organischen Mengenstrom (P) so bemißt, daß in (5) ein möglichst weitgehender Übergang des in besagtem organischen Mengenstrom (P) enthaltenen Polyamins in die wäßrige Phase (Q) erfolgt, die in der vorgeschalteten Extraktionsstufe (5) resultierende wäßrige Phase (Q) aus Mengenstrom (Y) und/oder Hilfsamin der Extraktionsstufe (6) zuführt und die in der vorgeschalteten Extraktionsstufe (5) anfallende an Polyamin verarmte organische Phase (R) zumindest teilweise der Extraktionsstufe (7) zuführt.

4. Verfahren gemäß einem oder mehreren der Ansprüche 1 bis 3, dadurch gekennzeichnet, daß man als Hilfsamin Anilin oder am Stickstoff alkylsubstituierte Aniline oder am aromatischen Kern alkylsubstituierte Aniline verwendet.

5. Verfahren gemäß einem oder mehreren der Ansprüche 1 bis 4, dadurch gekennzeichnet, daß man als Hilfsamin 2,6-Dimethylanilin verwendet.

6. Verfahren gemäß einem oder mehreren der Ansprüche 1 bis 4 ,dadurch gekennzeichnet, daß man als Hilfsamin 2-Methyl-6-ethylanilin verwendet.

7. Verfahren gemäß einem oder mehreren der Ansprüche 1 bis 4, dadurch gekennzeichnet, daß man als Hilfsamin N,N-Dimethylanilin verwendet.

8. Verfahren gemäß einem oder mehreren der Ansprüche 1 bis 4, dadurch gekennzeichnet, daß man als Hilfsamin Gemische verwendet, bestehend aus Anilin und/oder N- alkylsubstituierten Anilinen und/oder aus am aromatischen Kern alkylsubstituierten Anilinen.

9. Verfahren gemäß einem oder mehreren der Ansprüche 1 bis 4 und 7, dadurch gekennzeichnet, daß man als Hilfsamin Xylidingemische verwendet.

10. Verfahren gemäß einem oder mehreren der Ansprüche 1 bis 4 und 7, dadurch gekennzeichnet, daß man als Hilfsamin technische Alkylierungsgemische des Anilins und seiner Derivate verwendet.

11. Verfahren gemäß einem oder mehreren der Ansprüche 1 bis 10, dadurch gekennzeichnet, daß man als Polyamingemisch der Diphenylmethanreihe ein Polyamingemisch verwendet, wie es bei der säurekatalysierten Anilin/Formaldehyd-Kondensation anfällt.

12. Verfahren zur Herstellung aromatischer Polyisocyanatgemische, durch ein Verfahren enthaltend die folgenden Verfahrensstufen:
A) Herstellung aromatischer Polyamingemische der Diphenylmethanreihe gemäß den Ansprüchen 1-10,
B) Umwandlung der erhaltenen aromatischen Polyamingemische der Diphenylmethanreihe der Stufe (A) in die entsprechenden aromatischen Polyisocyanatgemische.

13. Verfahren zur Herstellung kernhydrierter Polyamine, Vernetzer und Epoxidhärter durch ein Verfahren enthaltend die folgenden Verfahrensstufen:
A) Herstellung aromatischer Polyamingemische der Diphenylmethanreihe gemäß den Ansprüchen 1-10,
B) Umwandlung der erhaltenen aromatischen Polyamingemische der Diphenylmethanreihe der Stufe (A) in die entsprechenden kernhydrieter Polyamine oder Vernetzen und Epoxidhärter.

14. Verfahren zur Herstellung von Polyurethankunststoffen durch ein Verfahren enthaltend die folgenden Verfahrensstufen:
A) Herstellung aromatischer Polyamingemische der Diphenylmethanreihe gemäß den Ansprüchen 1 - 10,
B) Umwandlung der erhaltenen aromatischen Polyamingemische der Diphenylmethanreihe der Stufe (A) in die entsprechenden Polyurethankunststoffe.

## Claims

1. Process for the fractionation and purification of mixtures of aromatic polyamines of the diphenylmethane series, characterized in that
a) the starting polyamine mixture (A) is partitioned in a two-phase system consisting of (i) a hydrophobic solvent phase (B) consisting substantially of aromatic auxiliary amine, which is sparingly soluble in water and whose boiling point under normal pressure is at least 20°C below the boiling point of the lowest-boiling component of the starting mixture, and (ii) an aqueous phase (C) consisting substantially of an aqueous solution of a strong acid and optionally auxiliary amine present at least partially in the salt form, with the aid of an extraction stage (7) operating according to the countercurrent principle, and with thorough mixing of the phases, the starting polyamine mixture being introduced into the extraction stage (7) via the aqueous phase (C), with the proviso that, in this two-phase system, the amine equivalents introduced into the streams (A), (B) and (C) are always in excess of the number of acid equivalents introduced into the stream (C), and the organic phase (D) leaving this extraction stage is separated,
b) at least partially via an intermediate extraction stage (6) and/or
c) with separation of a partial stream before or after passage through the extraction stage (6), and recycling of the separated partial stream, via an upstream extraction stage (5), at least partially into the extraction stage (7),
d) after passage through a washing stage and/or neutralization stage (10), in a distillation stage (11), into a distillate fraction, consisting substantially of auxiliary amine, and a first polyamine fraction, obtained as the distillation residue (G),
e) the aqueous phase (H) leaving the extraction stage (7) is introduced into a neutralization stage (8), the acid contained in the aqueous phase is neutralized with bases, and the resulting product is then mechanically separated, in a phase separation step, into an aqueous phase, containing the acid in the form of its neutral salts, and an organic phase, containing substantially polyamine and auxiliary amine, and
f) the organic phase (J) obtained in the neutralization stage (8) is optionally passed through a washing stage (9) and is at least partially worked up, in a distillation stage (12), into a distillate fraction (K), containing substantially auxiliary amine, and a second polyamine fraction, obtained as the distillation residue (L).

2. Process according to Claim 1, characterized in that
b) the organic phase (D) obtained in the extraction stage (7) is at least partially extracted in an intermediate extraction stage (6) in countercurrent with at least part of the aqueous acid (stream X), and/or extracted in countercurrent with all of the aqueous phase (Q) obtained in the upstream extraction stage (5), if present, the aqueous phase (N) resulting from the intermediate extraction stage (6) is fed into the extraction stage (7), and the organic phase (O) obtained in the intermediate extraction stage (6) is fed into the working-up stage (11).

3. Process according to Claim 1 or 2, characterized in that
c) a partial stream of the organic phase (D) leaving the extraction stage (7), and/or a partial stream of the organic phase (O) leaving the intermediate extraction stage (6), are separated off and, in an upstream extraction stage (5), reacted in one stage or extracted in several stages in countercurrent with at least part of the aqueous acid available as the stream (X), the organic stream (P) used in the extraction stage (5) is proportioned so that, in (5), the greatest possible amount of the polyamine contained in said organic stream (P) passes into the aqueous phase (Q), the aqueous phase (Q) resulting from the upstream extraction stage (5), consisting of the stream (Y) and/or auxiliary amine, is fed into the extraction stage (6), and the polyamine-depleted organic phase (R) obtained in the upstream extraction stage (5) is at least partially fed into the extraction stage (7).

4. Process according to one or more of Claims 1 to 3, characterized in that the auxiliary amine used is aniline, anilines alkyl-substituted on the nitrogen, or anilines alkyl-substituted on the aromatic ring.

5. Process according to one or more of Claims 1 to 4, characterized in that the auxiliary amine used is 2,6-dimethylaniline.

6. Process according to one or more of Claims 1 to 4, characterized in that the auxiliary amine used is 2-methyl-6-ethylaniline.

7. Process according to one or more of Claims 1 to 4, characterized in that the auxiliary amine used is N,N-dimethylaniline.

8. Process according to one or more of Claims 1 to 4, characterized in that the auxiliary amine used is a mixture consisting of aniline and/or N-alkyl-substituted anilines and/or anilines alkyl-substituted on the aromatic ring.

9. Process according to one or more of Claims 1 to 4 and 7, characterized in that the auxiliary amine used is a xylidene mixture.

10. Process according to one or more of Claims 1 to 4 and 7, characterized in that the auxiliary amine used is a technical-grade alkylation mixture of aniline and derivatives thereof.

11. Process according to one or more of Claims 1 to 10, characterized in that the mixture of polyamines of the diphenylmethane series used is a polyamine mixture such as that obtained in the acid-catalyzed aniline/formaldehyde condensation.

12. Process for the preparation of mixtures of aromatic polyisocyanates by a process comprising the following steps:
A) preparation of mixtures of aromatic polyamines of the diphenylmethane series according to Claims 1 - 10, and
B) conversion of the resulting mixtures of aromatic polyamines of the diphenylmethane series of step A) to the corresponding mixtures of aromatic polyisocyanates.

13. Process for the preparation of ring-hydrogenated polyamines, crosslinking agents and epoxy hardeners by a process comprising the following steps:
A) preparation of mixtures of aromatic polyamines of the diphenylmethane series according to Claims 1 - 10, and
B) conversion of the resulting mixtures of aromatic polyamines of the diphenylmethane series of step A) to the corresponding ring-hydrogenated polyamines or crosslinking agents and epoxy hardeners.

14. Process for the manufacture of polyurethane plastics by a process comprising the following steps:
A) preparation of mixtures of aromatic polyamines of the diphenylmethane series according to Claims 1 - 10, and
B) conversion of the resulting mixtures of aromatic polyamines of the diphenylmethane series of step A) to the corresponding polyurethane plastics.

## Revendications

1. Procédé pour le fractionnement et la purification de mélanges de polyamines aromatiques de la série des diphénylméthanes, caractérisé en ce que
a) on répartit le mélange de polyamines de départ (A) en un système biphasique constitué par (i) une phase de solvant hydrophobe (B) qui est constituée essentiellement d'une amine auxiliaire aromatique qui est peu soluble dans l'eau et qui présente, sous pression normale, un point d'ébullition d'au moins 20°C inférieur au point d'ébullition du composant du mélange de départ possédant le point d'ébullition le plus bas, et par (ii) une phase aqueuse (C) constituée essentiellement d'une solution aqueuse d'un acide fort et, le cas échéant, d'une amine auxiliaire présente au moins en partié sous forme saline, en faisant appel a une étape d'extraction (7) travaillant conformément au principe d'un écoulement à contre-courant, avec mélange intime des phases, de telle sorte que l'on introduit le mélange de polyamines de départ via la phase aqueuse (C) dans l'étape d'extraction (7), avec cette mesure que, dans ce système biphasique, les équivalents d'amine introduits dans les courants massiques (A), (B) et (C) dépassent toujours le nombre des équivalents d'acide introduits dans le courant massique (C), et on sépare la phase organique (D) quittant cette étape d'extraction,
b) au moins en partie via une étape d'extraction (6) intercalée et/ou
c) en séparant un courant partiel avant ou après son passage par l'étape d'extraction (6) et en recyclant le courant partiel séparé, via une étape d'extraction (5) montée en amont, au moins en partie dans l'étape d'extraction (7),
d) après son passage par une étape de lavage et/ou par une étape de neutralisation (10), dans une étape de distillation (11), en une fraction de distillat constituée essentiellement de l'amine auxiliaire et en une première fraction de polyamine que l'on obtient sous forme de résidu de distillation (G);
e) on guide dans une étape de neutralisation (8) la phase aqueuse (H) quittant l'étape d'extraction (7), on neutralise avec des bases l'acide contenu dans la phase aqueuse, et on la sépare ensuite par voie mécanique, dans une étape de séparation de phase, en une phase aqueuse contenant l'acide sous forme de ses sels neutres et en une phase organique contenant essentiellement la polyamine et l'amine auxiliaire; et
f) on traite la phase organique (J) obtenue dans l'étape de neutralisation (8), le cas échéant après son passage par une étape de lavage (9), au moins en partie dans une étape de distillation (12) pour obtenir une fraction de distillat (K) contenant essentiellement l'amine auxiliaire et une seconde fraction de polyamine obtenue sous forme de résidu de distillation (L).

2. Procédé selon la revendication 1, caractérisé en ce que
b) on extrait la phase organique (D) obtenue à l'étape d'extraction (7) au moins en partie dans une étape d'extraction intercalée (6) à contre-courant avec au moins une quantité partielle de l'acide aqueux (courant massique X) et/ou on l'extrait a contre-courant avec la quantité totale de la phase aqueuse (Q) obtenue dans l'étape d'extraction (5) le cas échéant montée en amont; on achemine à l'étape d'extraction (7) la phase aqueuse résultante (N) obtenue dans l'étape d'extraction intercalée (6); et on achemine à l'étape de traitement (11) la phase organique (O) obtenue dans l'étape d'extraction intercalée (6).

3. Procédé selon la revendication 1 ou 2, caractérisé en ce que
c) on sépare un courant partiel de la phase organique (D) quittant l'étape d'extraction (7) et/ou un courant partiel de la phase organique (O) quittant l'étape d'extraction intercalée (6) et on le fait réagir en une seule étape, dans une étape d'extraction (5) montée en amont, avec au moins une quantité partielle de l'acide aqueux disponible sous forme de courant massique (X); on l'extrait en plusieurs étapes à contre-courant; on dimensionne le courant massique organique (P) mis en oeuvre dans l'étape d'extraction (5) de telle sorte que l'on obtienne dans (5) un transfert le plus étendu possible de la polyamine contenue dans le courant massique organique (P) en question, à la phase aqueuse (Q); on achemine à l'étape d'extraction (6) la phase aqueuse résultante (Q) obtenue à l'étape d'extraction (5) montée en amont, provenant du courant massique (Y) et/ou l'amine auxiliaire; et on achemine la phase organique (R) appauvrie en polyamine, que l'on obtient dans l'étape d'extraction (5) montée en amont, au moins en partie, à l'étape d'extraction (7).

4. Procédé selon une ou plusieurs des revendications 1 à 3, caractérisé en ce qu'on utilise, à titre d'amine auxiliaire, l'aniline ou des anilines portant un ou plusieurs substituants alkyle identiques ou différents sur l'atome d'azote ou encore des anilines portant un ou plusieurs substituants alkyle identiques ou différents sur le noyau aromatique.

5. Procédé selon une ou plusieurs des revendications 1 à 4, caractérisé en ce qu'on utilise, à titre d'amine auxiliaire, la 2,6-diméthylaniline.

6. Procédé selon une ou plusieurs des revendications 1 à 4, caractérisé en ce qu'on utilise, à titre d'amine auxiliaire, la 2-méthyl-6-éthylaniline.

7. Procédé selon une ou plusieurs des revendications 1 à 4, caractérisé en ce qu'on utilise, à titre d'amine auxiliaire, la N,N-diméthylaniline.

8. Procédé selon une ou plusieurs des revendications 1 à 4, caractérisé en ce qu'on utilise, à titre d'amine auxiliaire, des mélanges constitués par de l'aniline et/ou par des anilines portant un ou plusieurs substituants alkyle identiques ou différents sur l'atome d'azote et/ou par des anilines portant un ou plusieurs substituants alkyle identiques ou différents sur le noyau aromatique.

9. Procédé selon une ou plusieurs des revendications 1 à 4 et 7, caractérisé en ce qu'on utilise, à titre d'amine auxiliaire, des mélanges de xylidines.

10. Procédé selon une ou plusieurs des revendications 1 à 4 et 7, caractérisé en ce qu'on utilise, à titre d'amine auxiliaire, des mélanges d'alkylation techniques de l'aniline et de ses dérivés.

11. Procédé selon une ou plusieurs des revendications 1 à 10, caractérisé en ce qu'on utilise, à titre de mélanges de polyamines de la série des diphénylméthanes, un mélange de polyamines tel qu'on l'obtient lors de la condensation d'aniline-formaldéhyde catalysée par un acide.

12. Procédé pour la préparation de mélanges de polyisocyanates aromatiques via un procédé contenant les étapes opératoires ci-après:
A) préparation de mélanges de polyamines aromatiques de la série des diphénylméthanes selon les revendications 1 - 10,
B) transformation des mélanges de polyamines aromatiques obtenues de la série des diphénylméthanes de l'étape (A) en mélanges de polyisocyanates aromatiques correspondants.

13. Procédé pour la préparation de polyamines hydrogénées au noyau, d'agents de réticulation et de durcisseurs époxy via un procédé contenant les étapes opératoires ci-après:
A) préparation de mélanges de polyamines aromatiques de la série des diphénylméthanes selon les revendications 1 - 10,
B) transformation des mélanges de polyamines aromatiques obtenues de la série des diphénylméthanes de l'étape (A) en polyamines correspondantes hydrogénées au noyau ou encore en agents de réticulation et en durcisseurs époxy correspondants.

14. Procédé pour la préparation de matières synthétiques de polyuréthanne via un procédé contenant les étapes opératoires ci-après:
A) préparation de mélanges de polyamines aromatiques de la série des diphénylméthanes selon les revendications 1 - 10,
B) transformation des mélanges de polyamines aromatiques obtenues de la série des diphénylméthanes de l'étape (A) en matières synthétiques de polyuréthanne correspondantes.
